Europäisches Patentamt

European Patent Office

Office européen des brevets

(19)

(11) Publication number: **0 443 036 B1**

## (12) EUROPEAN PATENT SPECIFICATION

(45) Date of publication of patent specification :
**13.09.95 Bulletin 95/37**

(51) Int. Cl.[6] : **G01N 33/53,** G01N 33/96,
G01N 33/574

(21) Application number : **90913230.0**

(22) Date of filing : **07.09.90**

(86) International application number :
**PCT/JP90/01148**

(87) International publication number :
**WO 91/03736 21.03.91 Gazette 91/07**

(54) **METHOD OF DETERMINING KINETICS OF IMMUNITY.**

(30) Priority : **08.09.89 JP 234484/89**

(43) Date of publication of application :
**28.08.91 Bulletin 91/35**

(45) Publication of the grant of the patent :
**13.09.95 Bulletin 95/37**

(84) Designated Contracting States :
**DE FR GB**

(56) References cited :
**JP-A-63 502 695
BIOLOGICAL ABSTRACTS vol. 81, no. 3 , 1986,
Philadelphia, PA, US; abstract no. 25264, page
643 ;
INSPECTION AND TECHNOLOGY, extra edi-
tion, Immunochemical Inspection Method,
Volume 16, No. 7, June 1988 (Hitoshi Kono and
others), "Present Status of Cellular Immunity
Inspection" P. 902-904.**

(56) References cited :
**J. Clin. Oncol., Vol. 7, No. 7 (1989)
"Immunologec Determinants of Head and
neck Cancer Response to Incution
Chemotherapy", SCHANTY S P et al., p.
857-864.
J. Clin, Immunol., Vol. 8, No. 2, (1988)
"Methionine Enkephalin as Immunomodulator
Therapy in Human Immunodeficiency virus
infections clinical and immunological effects"
ZUNICK K M et al., p. 95-102.**

(73) Proprietor : **YAMAUCHI, Tamio
2-2-29, Hazawa
Nerima-ku, Tokyo 176 (JP)**

(72) Inventor : **YAMAUCHI, Tamio
2-2-29, Hazawa
Nerima-ku, Tokyo 176 (JP)**

(74) Representative : **VOSSIUS & PARTNER
Postfach 86 07 67
D-81634 München (DE)**

EP 0 443 036 B1

## Description

This invention is a method of measuring cellular immunokinetics based upon the recognition mechanism of various antigens in human beings.

With the recent development of technology in the anticancer immunotherapy, a wide variety of remedies have been developed and various therapeutic methods devised. However, the cellular immunity in relation to immunotherapy in humans has not yet been completely elucidated and has been grasped in the dark.

The inventor has made analysis and comparison to examine how effective and ineffective cases differ or what the immune state in normal healthy persons without past histories of malignant neoplasms like cancers or sarcomas is like, taking, as examples, patients with metastatic renal cancer who had undergone an aggressive interferon-alpha ($\alpha$-IFN) treatment to continuously monitor the kinetics of activated suppression inducer/helper, helper and suppression inducer T lymphocytes, natural killer (NK) cells; OKIa1 antigen positive lymphocytes and so on.

As a result, it has been found that by the multiple regression analysis (MRA), which is one of multivariate data analyses, for the correlation of monocyte with helper activated suppression inducer/helper T lymphocytes, new and important findings in respect of cellular immunity, especially cancer immunokinetics, are obtained.

More specifically, it has now been found that the above-mentioned analysis renders it possible to estimate the function of autologous tumor antigen recognition mechanism (ATARM), the possibility of progress from carcinogenesis to nosogenesis and the presence, state and details of malignant neoplasm and is accordingly useful for the detection of cancers, tumors etc. and can be utilized as indices for the efficacy of their treatments.

As for activated suppression inducer/helper, helper and suppression inducer T lymphocytes, natural killer cells and OKIa1 antigen positive lymphocytes these lymphocytes have only been measured individually and the mutual relationships among them have never been examined up to date. That is to say, it is the real state of things that the significance of the results of such measurement has never been made clear and that immunokinetics have never been grasped on the basis of such results.

The object of this invention is to provide a method of measuring immunokinetics, which permits immunokinetics of cellular immunity, especially cancer immunity, to be grasped in a straightforward manner, and further can serve as an index for the diagnosis, treatment, prognosis etc. of different diseases, in particular autoimmune diseases like bronchial asthma, atopic dermatitis etc. hereditary gene diseases causing abnormal immune states, immune from organ transplantation, viral diseases affecting cellular immunity like serum hepatitis, cancer etc.

The inventor has succeeded in the assessment of cellular immunokinetics, especially cancer immunokinetics, by the MRA, which is one of multivariate data analyses, of the counts in human blood samples of activated suppression inducer/helper, activated suppressor/cytotoxic, suppression inducer, helper, suppressor and cytotoxic T lymphocytes, CD3, CD4, CD8 and OKIa1 antigen positive lymphocytes, monocytes and natural killer cells.

Accordingly, the gist of this invention consists of the following 1-9:

1. A method of measuring immunokinetics through blood analysis, characterized by carrying out the following process steps (a) - (f) to assess the antigen recognition mechanism:

   a) The counts of monocyte, helper T lymphocytes and the subset of activated suppression inducer/helper T lymphocyte in blood samples are measured;

   b) The multiple regression analysis using monocytes as dependent variables and helper and the subset of activated suppression inducer/helper T lymphocytes as independent variables is carried out;

   c) The respective partial regression coefficients of helper and the subset of activated suppression inducer/helper T lymphocytes are determined and the significances are tested;

   d) The multiple correlation coefficient is determined and the significance is tested;

   e) The contributions of helper and the subset of activated suppression inducer/helper T lymphocytes are determined; and

   f) The correlations between monocytes helper T lymphocytes and the subset of activated suppression inducer/helper T lymphocytes are determined.

2. A method of measuring immunokinetics through blood analysis, characterized by carrying out the following process steps (a) - (f) to assess the suppressive function of natural killer cells on suppression inducer T lymphocytes:

   a) The counts of natural killer cells, suppression inducer T lymphocytes and the subset of activated suppression inducer/helper T lymphocytes in blood samples are measured;

   b) The multiple regression analysis using natural killer cells as dependent variables and suppression inducer and the subset of activated suppression inducer/helper T lymphocytes as independent vari-

EP 0 443 036 B1

ables is carried out;

c) The respective partial regression coefficients of suppression inducer and the subset of activated suppression inducer/helper T lymphocytes are determined and the significances are tested;

d) The multiple correlation coefficient is determined and the significance is tested;

e) The contributions of suppression inducer and the subset of activated suppression inducer/helper T lymphocytes are determined; and

f) The correlations between natural killer cells, suppression inducer and the subset of activated suppression inducer/helper T lymphocytes are determined.

3. A method of measuring immunokinetics through blood analysis, characterized by carrying out the following process steps (a) - (f) to assess the function of natural killer cells and monocytes against OKIa1 antigen positive lymphocytes.

a) The counts of OKIa1 antigen positive lymphocytes, natural killer cells and monocytes in blood samples are measured;

b) The multiple regression analysis using OKIa1 antigen positive lymphocytes as dependent variables and using natural killer cells and monocytes as independent variables is carried out;

c) The respective partial regression coefficients of natural killer cells and monocytes are determined and the significances are tested;

d) The multiple correlation coefficient is determined and the significance is tested;

e) The contributions of natural killer cells and monocytes are determined; and

f) The correlations between OKIa1 antigen positive lymphocytes natural killer cells and monocytes are determined.

4. A method of measuring immunokinetics through blood analysis, characterized by carrying out the following process steps (a) - (f) to assess the function of natural killer cells and monocytes on activated suppression inducer/helper and activated suppressor/cytotoxic T lymphocytes:

a) The counts of activated suppression inducer/helper and activated suppressor/cytotoxic T lymphocytes, natural killer cells and monocytes in blood samples are measured:

b) The multiple regression analysis using the sum of activated suppression inducer/helper and activated suppressor/cytotoxic T lymphocytes as dependent variables and natural killer cells and monocytes as independent variables is carried out;

c) The respective partial regression coefficients of natural killer cells and monocytes are determined and the significances are tested;

d) The multiple correlation coefficient is determined and the significance is tested;

e) The contributions of natural killer cells and monocytes are determined; and

f) The correlations of the sum of activated suppression inducer/helper and activated suppressor/cytotoxic T lymphocytes with natural killer cells and monocytes are determined.

5. A method of measuring immunokinetics through blood analysis, characterized by carrying out the following process steps (a) - (g) to assess the function of natural killer cells and monocytes on activated B lymphocytes.

a) The counts of monocytes natural killer cells, OKIa1 antigen positive lymphocytes, and the subset of activated suppression inducer/helper and the subset of activated suppressor/cytotoxic T lymphocytes in blood samples are measured;

b) Activated B lymphocytes count is calculated by subtracting from the count of OKIa1 antigen positive lymphocytes that of the subset of activated suppression inducer/helper T lymphocytes and that of the subset of activated suppressor/cytotoxic T lymphocytes;

c) The multiple regression analysis using activated B lymphocytes as dependent variable and natural killer cells and monocytes as independent variables is carried out;

d) The respective partial regression coefficients of natural killer cells and monocytes are determined and the significances are tested;

e) The multiple correlation coefficient is determined and the significance is tested;

f) The contributions of natural killer cells and monocytes are determined; and

g) The correlations of activated B lymphocytes with natural killer cells and monocytes are determined.

6. A method of measuring immunokinetics through blood analysis, characterized by carrying out the following process steps (a) - (d) to obtain standardized partial regression coefficients and multiple correlation coefficients and plotting the coefficients on coordinates to prepare graphs for assessment:

a) The counts of monocytes, natural killer cells OKIa1, antigen positive lymphocytes, helper and suppression inducer T lymphocytes and the subset of activated suppression inducer/helper T lymphocytes in blood samples are measured;

b) The standardized partial regression coefficients of helper and the subset of activated suppression inducer/helper T lymphocytes are determined by the multiple regression analysis, using monocytes as dependent variables and helper and the subset of activated suppression inducer/helper T lymphocytes as independent variables, to obtain the correlations of monocytes with helper and the subset of activated suppression inducer/helper T lymphocytes;

c) The standardized partial regression coefficients of suppression inducer and the subset of activated suppression inducer/helper T lymphocytes are determined by the multiple regression analysis using natural killer cells as dependent variables and suppression inducer and the subset of activated suppression inducer/helper T lymphocytes as independent variables to obtain the correlations of natural killer cells with suppression inducer and the subset of activated suppression inducer/helper T lymphocytes; and

d) The standardized partial regression coefficients of natural killer cells and monocytes are determined by the multiple regression analysis using OKIa1 antigen positive lymphocytes as dependent variables and natural killer cells and monocytes as independent variables to obtain the correlations of OKIa1 antigen positive lymphocytes with natural killer cells and monocytes.

7. A method of measuring immunokinetics through blood analysis, characterized carrying out the following process steps (a) - (f) to obtain standardized partial regression coefficients and multiple correlation coefficients and plotting the coefficients on coordinates to prepare graphs for assessment:

a) The counts of monocytes, natural killer cells, OKIa1 antigen positive lymphocytes, the subset of activated suppression inducer/helper and activated suppressor/cytotoxic T lymphocytes, and helper and suppression inducer T lymphocytes in blood samples are measured;

b) The standardized partial regression coefficients of helper and the subset of activated suppression inducer/helper T lymphocytes are determined by the multiple regression analysis using monocytes as dependent variables and helper and the subset of activated suppression inducer/helper T lymphocytes as independent variables to obtain the correlations of monocytes with helper and the subset of activated suppression inducer/helper T lymphocytes;

c) The standardized partial regression coefficients of suppression inducer and the subset of activated suppression inducer/helper T lymphocytes are determined by the multiple regression analysis using natural killer cells as dependent variables and suppression inducer and the subset of activated suppression inducer/helper T lymphocytes as independent variables to obtain the correlations of natural killer cells with suppression inducer and the subset of activated suppression inducer/helper T lymphocytes;

d) The standardized partial regression coefficients of natural killer cells and monocytes are determined by the multiple regression analysis using OKIa1 antigen positive lymphocytes as dependent variables and natural killer cells and monocytes as independent variables to obtain the correlations of OKIa1 antigen positive lymphocytes with natural killer cells and monocytes;

e) The standardized partial regression coefficients of natural killer cells and monocytes are determined by the multiple regression analysis using the sum of the subset of activated suppression inducer/helper and the subset of activated suppressor/cytotoxic T lymphocytes as dependent variables and natural killer cells and monocytes as independent variables to obtain the correlations of activated T lymphocytes with natural killer cells and monocytes; and

f) Activated B lymphocyte count is calculated by subtracting the counts of the subset of activated suppression inducer/helper and activated suppressor/cytotoxic T lymphocytes from the OKIa1 antigen positive lymphocyte count and the standardized partial regression coefficients of natural killer cells and monocytes are determined by the multiple regression analysis using activated B lymphocyte as dependent variable and natural killer cells and monocytes as independent variables to obtain the correlations of activated B lymphocytes with natural killer cells and monocytes.

8. A method of measuring and assessing immunokinetics through blood analysis, characterized by carrying out the following process steps (a) - (f) and assessing integrated T cell immunokinetics on the basis of the resultant T cell immunokinematograms:

a) The counts of CD3, CD4 and CD8 antigen positive lymphocytes, helper, suppression inducer, suppressor and cytotoxic T lymphocytes, the subset of activated suppression inducer/helper and the subset of activated suppressor/cytotoxic T lymphocytes, natural killer cells and monocytes in blood samples are measured;

b) The multiple regression analysis using the subset of activated suppressor/cytotoxic T lymphocytes as dependent variables and the subset of activated suppression inducer/helper, helper, suppression inducer, cytotoxic and suppressor T lymphocytes, CD3, CD4 and CD8 antigen positive lymphocytes, monocytes and natural killer cells as independent variables is carried out;

c) The respective contributions of the subset of activated suppression inducer/helper, helper, suppres-

EP 0 443 036 B1

sion inducer, cytotoxic and suppressor T lymphocytes, CD3, CD4 and CD8 antigen positive lymphocytes, natural killer cells and monocytes against the subset of activated suppressor/cytotoxic T lymphocytes is determined;

d) The contributions of activated suppression inducer and activated helper T lymphocytes are calculated by allotting the contribution of the subset of activated suppression inducer/helper T lymphocytes in proportion to the ratio between the contributions of suppression inducer and helper T lymphocytes;

e) The contributions of activated suppressor and cytotoxic T lymphocytes and calculated by allotting the contribution of the subset of activated suppressor/cytotoxic T lymphocytes obtained in step b) in proportion to the ratio between the contributions of suppressor and cytotoxic T lymphocytes; and

f) The respective contributions obtained in steps d) to e) are converted into circular areas, and the respective circular areas are arranged in accordance with the mutual relationships among activated helper, activated suppression inducer, activated suppressor and activated cytotoxic T lymphocytes to draw T cell immunokinematograms.

9. A method of measuring and assessing immunokinetics through blood analysis, characterized by carrying out the following process steps (a) - (g) and assessing integrated T cell immunokinetics on the basis of the resultant B cell immunokinematograms:

a) The counts of helper, suppression inducer, cytotoxic and suppressor T lymphocytes, the subset of activated suppression inducer/helper and the subset of activated suppressor/cytotoxic T lymphocytes, OKIa1 antigen positive lymphocytes, natural killer cells, monocytes, basophils, eosinophils and neutrophils in blood samples are measured;

b) Activated B lymphocytes count is calculated by subtracting from the OKIa1 antigen positive lymphocytes count the subset of activated suppression inducer/helper T lymphocytes and the subset of activated suppressor/cytotoxic T lymphocytes counts;

c) The multiple regression analysis using activated B lymphocyte as dependent variable and helper, suppression inducer, cytotoxic, suppressor, the subset of activated suppression inducer/helper and the subset of activated suppressor/cytotoxic T lymphocytes, natural killer cells, monocytes, basophils, eosinophils and neutrophils as independent variables is carried out;

d) The respective contributions of helper, suppression inducer, cytotoxic, suppressor, the subset of activated suppression inducer/helper and activated suppressor/cytotoxic T lymphocytes, natural killer cells, monocytes, basophils, eosinophils and neutrophils against activated B lymphocytes are determined;

e) The contributions of activated helper and activated suppression inducer T lymphocytes are calculated by allotting the contribution of the subset of activated suppression inducer/helper T lymphocytes in proportion to the ratio between the contributions of helper and suppression inducer T lymphocytes;

f) The contribution of the subset of activated suppressor/cytotoxic T lymphocytes obtained in step c) is allotted in proportion to the ratio between the contributions of suppressor and cytotoxic T lymphocytes to calculate the contributions of activated suppressor and activated cytotoxic T lymphocytes; and

g) The respective contributions calculated in steps d), e) and f) except for the contributions of cytotoxic and activated cytotoxic T lymphocytes are converted into circular areas, and the respective circular areas are arranged in accordance with their immunologic mutual relationships to draw the B cell immunokinematograms.

In the following explanations as will be seen later, activated suppression inducer/helper, activated suppressor/cytotoxic, helper, suppression inducer, cytotoxic, suppressor, activated suppression inducer, activated helper, activated suppressor and activated cytotoxic T lymphocytes and OKIa1 antigen positive lymphocytes may be abbreviated to Act.si/h T, Act.s/c T, Th, Tsi, Tc, Ts, Act.Tsi, Act.Th, Act.Ts, Act.Tc and OKIa1$^+$, respectively.

In carrying out the peripheral lymphocyte measurement, blood samples are conditioned in the usual way by addition heparin, EDTA or ACD solution as anticoagulant, and then subjected to flowcytometry.

Measurements of peripheral lymphocytes are carried out preferably with monoclonal antibodies (MoAbs) corresponding to the respective subpopulations. Examples of MoAbs which may be used include Leu-series (Becton-Dickinson Immunocytometry Systems), OK-series (Ortho Diagnostic Systems Inc.) and T-series (Coulter Corp.). There is no restriction in the choice of MoAbs, provided that they belong to the same cluster numbers of differentiation (CD). Such measurements are carried out preferably by the fluorescent antibody technique using the single or two-color flowcytometry.

In carrying out the measurement of monocyte count, blood samples are usually applied to a full-automatic hemocytometer to count all monocytes therein. Where the monocyte count is too few to carry out such measurement, however, it is measured microscopically by the use of usual blood smears. In that case, not less than 300, preferably not less than 500, white blood cells are counted, out of which the monocyte count is calculated.

After the above-mentioned measurements of peripheral lymphocyte subpopulations and monocytes, one or more, or preferably all, of the following multiple regression analyses (MRAs) (A) to (E) are carried out.

(A) The multiple regression analysis (MRA) using monocytes as dependent variables and helper and the subset of activated suppression inducer/helper T lymphocytes as independent variables is carried out.

(B) The MRA using natural killer cells as dependent variables and suppression inducer and the subset of activated suppression inducer/helper T lymphocytes as independent variables is carried out.

(C) The MRA using OKIa1 antigen positive lymphocytes as dependent variables and natural killer cells and monocytes as independent variables is carried out .

(D) The MRA using the sum of activated suppression inducer/helper and activated suppressor/cytotoxic T lymphocytes counts as dependent variables and natural killer cells and monocytes as independent variables is carried out.

(E) Activated B lymphocyte count is calculated by subtracting from OKIa1 antigen positive lymphocyte count the sum of the subset of activated suppression inducer/helper and the subset of activated suppressor/cytotoxic T lymphocytes counts, and then the MRA using the activated B lymphocytes as dependent variables and natural killer cells and monocytes as independent variables is carried out.

The combination of the above-mentioned (A), (B) and (C), the combination of (A), (B) and (D) and the combination of (A), (B) and (E) are called the immunologic three-factor set, the T-cell immunologic three-factor set and the B-cell immunologic three-factor set, respectively, for the sake of convenience.

The respective correlations in (A) to (E) are indicative of special relations namely (A): recognition mechanisms for autologous tumor antigen etc.; (B): function of natural killer cells; (C): cooperative function between activated lymphocytes and monocytes and natural killer cells; (D): cooperative function between activated T lymphocytes, and monocytes and natural killer cells and (E): cooperative function between activated B lymphocytes, and monocytes and natural killer cells, respectively. Even with the determination of at least one of correlations (A) - (E), the immunologic assessment is possible to a certain degree.

These correlations (A) - (E) are also specific to the blood sample used and therefore serve for example as indices for the discrimination between abnormality or normality, and for the therapeutic planning.

In determining such correlations, it is desirable that the respective contributions of peripheral lymphocyte subpopulations are used to draw cell immunokinematograms, for example, by converting them into the corresponding circular areas. Since monocytes infiltrate within and/or around the cancer tissue and thereafter functionally differentiate through macrophages (Mφ) into activated Mφ, the MRAs can be carried out by substituting such activated Mφ count for monocyte count, if it is possible to measure the activated Mφ count. In the cell immunokinematograms shown as examples of this invention, therefore, the contribution of activated Mφ is expressed in terms of that of monocytes for the sake of convenience.

In accordance with this invention, it becomes possible not only to easily grasp immunokinetics, but also to predict whether the patient will relapse or not in future, for example, by comparing the results of peripheral subpopulation determination in an unknown case with the corresponding data obtained with known cases or with the time-course data obtained with the same patient, whereby the diagnosis of diseases in relation to immunity against cancer or the like is facilitated and indices for therapeutic planning against cancer or the like become available.

In accordance with the invention by measuring the above-mentioned specific lymphocyte subpopulations and monocyte counts; carrying out MRAs (A) - (E) mentioned above; determining the standardized partial regression coefficients (SPRCs); and determining correlations between the respective factors as dependent variable and the respective factors as independent variable, it is possible, for example; to rapidly grasp patterns of immunokinetics in various malignancies. That is, by determining beforehand the immunokinetic patterns of normal control group, which may be represented, for example, in form of graph and comparing the patient's patterns of immunokinetics with the immunokinetic patterns of normal control group, it is possible to make a diagnosis of cancer and the like.

Different methods may be mentioned to determine such correlations: the respective multiple correlation coefficients (MCCs) obtained by MRAs (A) to (E) are plotted on three-dimensional coordinates to draw immunokinetic graphs, rader chart graphs or stack-bar graphs and so on. Furthermore the respective standardized partial regression coefficients (SPRCs) of independent variables obtained by MRAs (A) to (E) are plotted on two- or three-dimensional coordinates to draw patterns of triangle or triangular prism. Besides these two- or three-dimensional representations, it is also possible to draw four-dimensionally represented graphs as by addition of the factor of time, significance, contribution etc.

In making these assessments, various methods, for example, (1) the comparison between disease and normal control groups or (2) the serial and time-dependent comparison in the same patient, may be adopted.

Furthermore, according to the present invention, it is possible to assess integrated immunokinetics by drawing cellular immunokinematograms as mentioned in 8. and 9. above.

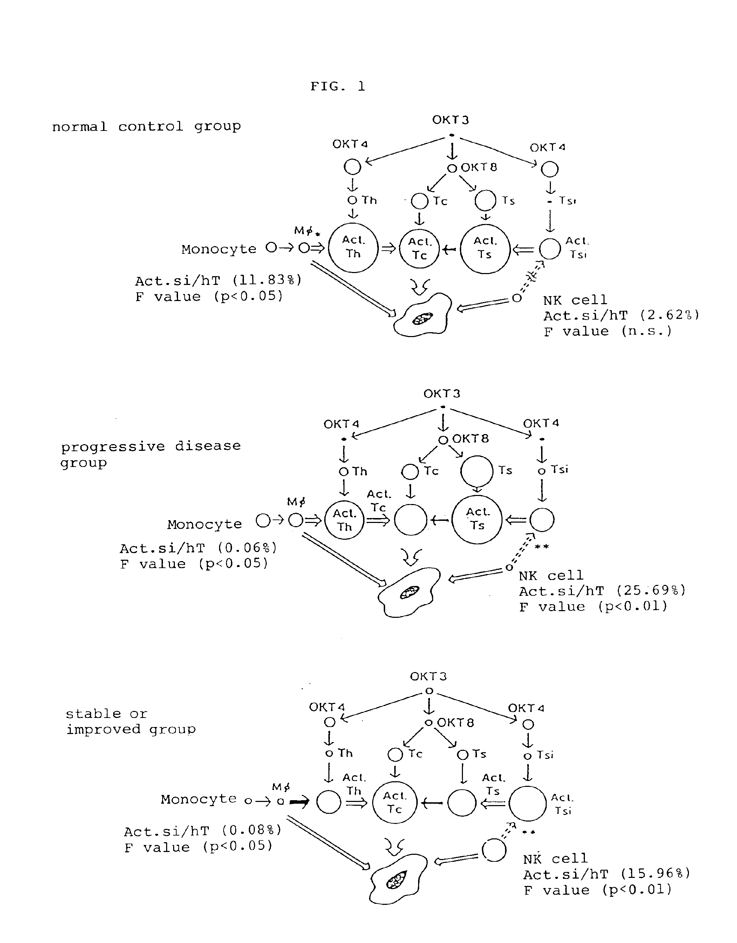

The cellular immunokinematograms in 8. and 9. above are T cell and B cell immunokinematograms, respectively. Taking as examples Figures 1, 2 and 7, representations, as well as their meanings in terms of the monocytes →·Act.Th and natural killer cells →·Act.Tsi processes, in the T cell immunokinematogram will be explained below:

=⇒ : There is no significant correlation in the MRA between monocytes and helpers (Th and Act.si/h T). It immunologically means that the antigen recognition mechanism is impaired, hypofunctional or non-functioning.

⇒ : In the same MRA as the above-mentioned, there is achieved for the first time a significance correlation therebetween (p<0.05) and the partial regression coefficient (PRC) of Act.si/h T must always be positive, because both the precursor Th and its active type, namely Act.si/h T, must be positively involved in the functional promotion of monocytes and especially in view of the fact that the active types performs the more strong function. It immunologically indicates that the antigen recognition mechanism are somewhat hyperfunctional.

⁺⇒ : In the MRA, monocytes achieves a significant correlation (p<0.05) only with and the PRC of Act.si/h T is always positive. It immunologically is indicative of a moderately hyperfunctional state of the antigen recognition mechanism.

⁺⁺⇒ : In the MRA, monocytes achieves a significant correlation (p<0.01) only with Act.si/h T and the PRC likewise is positive. It immunologically is indicative of an ultra-hyperfunctional state.

➡ : In the MRA, monocytes barely achieves a significant correlation (p<0.05) only with both of Th and Act.si/h T 0.05 of P-value and the PRC of Act.si/h T is negative. It immunologically indicates a suppression of the differentiation from monocytes to macrophages (Mø), especially a suppressive function of Mø against helpers, namely a suppressive function on the antigen recognition mechanism.

⁺➡ : In the MRA, monocytes achieves a significant correlation (p<0.05) only with Act.si/h T but its PRC is negative. It immunologically means a moderate hyperfunction of suppressive Mø, namely a moderate suppression of the antigen recognition mechanism.

⁺⁺➡ : In the MRA, monocyte achieves a significant correlation (p<0.01) only with Act.si/h T and its PRC is negative. It immunologically means an extreme suppression of the antigen recognition mechanism.

=⇢ : There is no significant correlation in the MRA between natural killer cells and suppression inducers (Tsi, Act.si/h T), and it immunologically means an impaired, hypofunctional or non-functioning suppression against Act.Tsi. Because these correlations are the measurement of the suppressive function of natural killer cells against Act.Tsi, this symbol was used in all cases where the PRC of Act.si/h T is negative regardless of whether significant or not.

===> : In the MRA, natural killer cells achieves a significant correlation (p<0.05) only with both Tsi and Act.si/h T and the PRC of Act.si/h T is positive. It immunologically means a suppressive function against Act.Tsi.

⁺⇢ : In the MRA between natural killer cells, and Tsi and Act.si/h T, natural killer cells achieve a significant correlation (p<0.05) only with Act.si/h T and the PRC of Act.si/h T is positive. It immunologically means a moderately suppressive function of NK cell.

⁺⁺⇢ : In the MRA between natural killer cells, and Tsi and Act.si/h T, natural killer cells achieve a significant correlation (p<0.01) only with Act.si/h T and the PRC of Act.si/h T is positive. It immunologically means an extremely suppressive function of natural killer cells.

In the following will be explained how to calculate activated B lymphocyte count, with reference to B-cell immunokinematograms obtained by 9. above.

OKIa1 antigen positive lymphocyte count is measured by single-color flow cytometry, where granulocytes, namely neutrophils, eosinophils and basophils, monocytes and lymphocytes are automatically separated. In carrying the measurement, the gate is set so that only lymphocyte count is obtained. The ratio of lymphocyte positive when stained with OKIa1 MoAb is automatically measured on a flow cytometer. Therefore, almost all of the OKIa1 antigen positive cells are lymphocytes.

The OKIa1 antigen positive cells are basically expressed as T and B lymphocytes, a part of natural killer cells and monocytes but monocytes are completely excluded because the gate for monocytes is off at the measurement of OKIa1 antigen positive lymphocytes. The problem is the partly-included natural killer cells, but their ratio is supposed to be low and therefore may be neglected for the purpose of statistic treatment. Thus, it is supposed that the majority of the OKIa1 antigen positive cells are T and B cells. The lymphocytes expressed as the OKIa1 antigen are considered to be activated lymphocytes and the OKIa1 antigen may be taken as being almost identical to HLA-DR, a major histocompatibility antigen class II. Therefore, activated T lymphocytes

EP 0 443 036 B1

(Act.T) count is calculated as Act.si/h T count plus Act.s/c T count. Activated B lymphocytes (Act.B) count is calculated as OKIa1 antigen positive lymphocytes count minus Act.T count. This is because the lymphocytes consist only of the two types, i.e. T and B lymphocytes.

For the strict determination of activated B lymphocytes count, MoAbs directed to several other kinds of B lymphocytes should be used in staining, which, however, will increase cost as well as the time and blood sample volume required for investigation. Therefore, from the view point of cost and complexity as well as from clinical practicality, OKIa1 antigen positive lymphocytes count minus activated T lymphocytes count, both counts obtained upon the measurement of T cell immunity, are taken, for the sake of convenience, as measured values for activated B lymphocytes Whenever, although rare, the calculated value of Act.B count becomes minus, the count of Act.B is regarded as zero. After those procedures, B cell immunokinematograms can be drawn.

The assessment of B cell immunity is useful and convenient for the understanding, for example, of immunities relating to the cellular system producing antibodies, as well as immunities involved in allergy and neutrophil-based immunities involved in bacterial infections.

The concrete methodology will now be explained below. In the same manner as described above for T cell immunity, the MRA using Act.B as dependent variablesand monocytes, Th, Tsi, Act.si/h T, Tc, Ts, Act.s/c T, natural killer cells, basophils, eosinophils and neutrophils as independent variables is carried out and the contribution of each independent variable is determined. As in the case of T cell immunity, based on the immunologic relationships with Act.B, the respective contributions converted into the corresponding circular areas are arranged. In carrying out this, the contributions of Act.Th, Act.Ts and Act.Tsi are determined not directly but by dividing the contributions of Act.si/hT and Act.s/cT in accordance with ratio of their precursors, i.e. Th, Tsi, Tc and Ts: Act.Th (%) = Act.si/h T x (Th ÷ (Th + Tsi)}, Act.Tsi (%) = Act.si/h T x {Tsi ÷ (Th + Tsi)}, and Act.Ts (%) = Act.s/cT x {Ts ÷ (Tc + Ts)}.

As for the relations in terms of the monocytes → Act.Th and natural killer cells →·Act.Tsi processes, the MRAs carried out for drawing T cell immunokinematograms and their results are adopted. Besides them, the relations between Act.B and helpers (Th and Act.si/h T) are assessed by the MRA using Act.B as dependent variables and Th and Act.si/h T as independent variables, and those between neutrophils and helpers, between eosinophils and helpers and between basophils and helpers, are assessed by the MRAs using neutrophils, eosinophils and basophils, respectively, as dependent variables and Th and Act.si/h T as independent variables.

According to these MRAs, it is possible, while drawing B cell immunokinematograms, to express the degree of the respective mutual relationships with concrete figures. The figures (in %) used are expressed in terms of the contribution of Act.si/hT in the respective MRAs, as is the case with the monocytes → Act.Th and natural killer cells → Act.Tsi processes. Furthermore, the symbols in these immunokinematograms are used to mean the following:

⇒ : There is no significance in the MRA and no immunologic hyperfunction is detected.

⇒ : In the MRA, there is a significant correlation (p<0.05 by f-test) only with both Th and Act.si/h T and the PRC of Act.si/h T must be positive. If the PRC of Act.si/h T is negative regardless of there being such significant correlation with both of Th and Act.si/h T, the symbol ⇒ must be used because both of precursors Th and Act.si/h T including its active type must be positively involved in the immunologic promotion. It means some degree of hyperfunction immunologically.

⇒ : In the MRA, there is significant correlation (p<0.05) only with Act.si/h T. It immunologically means a hyperfunction.

⇒ : In the MRA, there is a significant correlation (p<0.01) only with Act.si/h T. It immunologically means an extreme hyperfunction.

In the accompanying drawings, these four patterns of symbol will be shown.

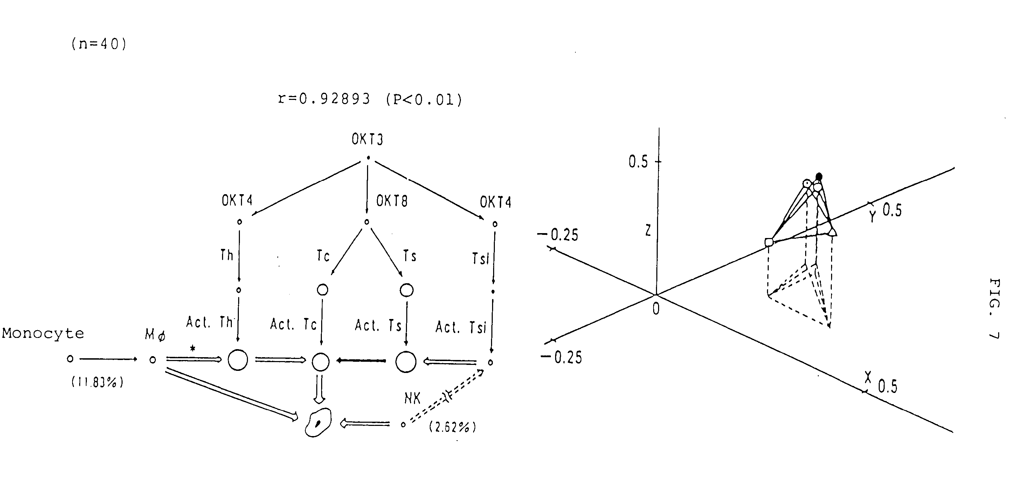

Thus, Figure 7 is taken as an example to explain cellular immunokinematograms and immunologic three-factor sets.

In the left of Figure 7, a T cell immunokinematogram is shown and the abbreviations and symbols therein are used to mean the following: → : differentiation and maturation; ⇒ : establishment of antigen recognition mechanism (p<0.05 by f-test); ⇒ : promotion or acceleration, ⟵ : suppression; ⇢ : no suppressive function of natural killer cells; ⇒ : cytotoxicity; ● : 0.1 % ≧ of contribution; ( %) : the contribution of activated suppression inducer/helper T cells versus monocytes or natural killer cells, γ: multiple correlation coefficient.

In the right of Figure 7, the immunologic three-factor sets are shown and the abbreviations and symbols therein are used to mean the following: Δ : The relation of monocyte versus helper and activated suppression

8

inducer/helper T lymphocytes: □ : The relation of natural killer cells versus suppression inducer and activated suppression inducer/helper T lymphocytes; ● : The relation of OKIa1 antigen positive lymphocytes versus monocytes and natural killer cells, ○ : The relation of activated T lymphocytes versus monocytes and natural killer cells; and ⊙ : The relation of activated B lymphocytes versus monocytes and natural killer cells.

The invention will be explained in more detail with reference to some examples.

[Examples]

Example 1

Measurement of Cellular Immunokinetics Based upon Autologous Tumor Antigen Recognition Mechanism in Metastatic Renal Cancer Patients.

MATERIALS

Twenty-four patients admitted and treated in the Urological Department of the Cancer Institute Hospital from June 1983 to December 1988 were used. Three cases among them had been treated by other clinics initially, in respect of which the detailed information on the staging and their histological diagnoses at the initial examination were not available. The staging and histological grading were conducted according to the criteria of the General Rule for Clinical and Pathological Studies on Renal Cell Carcinoma (The Japanese Urological Association, The Japanese Pathological Society and Japan Radiological Society). The majority of patients had metastases mostly to lungs and bones.

All of them were treated with human lymphoblastoid interferon (HLBI: Sumiferon®, Sumitomo Pharmaceutical Co., Ltd., Osaka, Japan). As a rule HLBI was dosed everyday intramuscularly starting with a dose of 3 million units, which were increased to 6, 9 and 12 million units, respectively, every week, and the maximal tolerable dose was maintained twice weekly. Some patients had the maintenance dose everyday, or 3 to 5 times weekly. In some cases, the radiotherapy and surgical resection were applied to the metastatic site concomitantly. Further, as oral anticancer agent, UFT (Tegafur/Uracil formulation, Taiho Yakuhin Kogyo, Co., Ltd. JAPAN) or doxifluridine (5'-DFUR: Furtulon®, Nippon Roche Co., Ltd., Tokyo, Japan) was used combinedly. Further, as intensive chemotherapy vinblastine and adriamycin were additionally dosed to 3 cases. Arterial embolization was performed at the primary lesion or local recurrence for a case with T4 of local 'extension inoperable together with metastases to the lung and liver, or another case with local recurrence 10 years after operation of the primary lesion and metastasis to the lung.

In addition to the treatment with HLBI, OK-432 (Picibanil®, Chugai Pharmaceutical Co., Ltd., Tokyo, Japan) was given as another BRMs in some cases, and, gene re-combinated $\gamma$-interferon ($\gamma$-IFN: supplied by Daiichi Seiyaku Co., Ltd., Tokyo, Japan), or $N^2$-(N-acetylmuramyl-L-alanyl-D-isoglutamyl)-$N^2$-stearoyl-L-lysine (MDP-Lys (L18): supplied by Daiichi Seiyaku Co., Ltd.), a derivative of muramyl dipeptide which is a component of the cell wall skeleton in tubercle bacillus, was also additionally applied in some cases. OK-432 was given subcutaneously everyday starting with 0.5 KE (Klinische Einheit) and increasing doses every 3 days to 1,2 and 3 KE, respectively and maintained at 3 KE twice weekly. MDP-Lys (L18) was maintained by subcutaneous injection at 200 mcg once weekly.

From the outset of the treatment with HLBI, peripheral lymphocyte subsets were determined with the passage of time, and as a rule a determination was always made at the addition of further treatment or at the change of treatment. Further, the determination was performed upon sufficient recovery of the bone marrow function, where that was strongly suppressed due to intensive chemotherapy. The therapeutic effect was evaluated according to the criteria for cancer chemocherapeutic evaluation of the Ministry of Health and Welfare. The subsets were measured simultaneously with the evaluation of clinical effects and when the lesions were unchanged or decreased their results were classified as the stable or improved group, and those measuring an increase of tumor size by more than 25% or occurrence of new lesion were classified as the progressive disease (PD) group. When evaluated once as PD, until the next time of evaluation of the lesion having increased by more than 25% and/or a new lesion having occurred again, the results of subsets measured during that time were classified as the stable or improved group. As a rule, the measurement of subsets was performed monthly and when the lesion is stabilized, once in 2 or 3 months.

Meanwhile, as the normal control, peripheral lymphocyte subsets of 40 healthy volunteers without history of malignant neoplasm such as cancer, in their age ranging from 31 o 78 years, 55.4 ± 13.0 years (mean ± S.D.), consisting of 17 females and 23 males, were measured.

DETERMINATION OF PERIPHERAL LYMPHOCYTE SUBSETS AND MONOCYTES

As a rule, lymphocytes were isolated from the heparinized blood collected out of the cubital vein. Upon collection and removal of red blood cells with a defined hemolytic agent, lymphocytes were stained with flurorescein isothiocyanate - or phycoerythrin-labelled monoclonal antibody - and then determined by the single-color flow cytometry (Spectrum III, Ortho Diagnostic Systems Inc., New Jersey, USA) and the two-color flow cytometry (FACS analyzer· Consort 30, Becton Dickinson Immunocytometry Systems, Mountain View, C.A.).

Lymphocytes positive to OKT3, OKT4, OKT8 and OKIa1 monoclonal antibodies (Ortho Diagnostic Systems Inc.) were determined on a single-color flow cytometer, and cells positive to $Leu3a^+ \cdot Leu8^-$ (helper T lymphocytes: Th), $Leu3a^+ \cdot Leu8a^+$ (suppression inducer T lymphocytes: Tsi), $Leu2a^+ \cdot Leu15^-$ (cytotoxic T lymphocytes: Tc), $Leu2a^+ \cdot Leu15^+$ (suppressor T lymphocytes: Ts), $Leu7^- \cdot Leu11c^+$ (natural killer cells: NK cells), $Leu3a^+ \cdot HLA\text{-}DR^+$ (activated suppression inducer/helper T lymphocytes: Act.si/h T) and $Leu2a^+ \cdot HLA\text{-}DR^+$ (activated suppressor/cytotoxic T lymphocytes Act.s/c T), were determined on a two-color flow cytometer, using monoclonal antibodies against Leu and HLA-DR manufactured by Becton Dickson Immunocytometry Systems.

Lymphocytes and monocytes in the peripheral blood were counted on a full-automatic hemocytometer (Coulter Electronics Corporation, Florida, USA). Meanwhile, when lymphocytes and monocytes were too few to count in the peripheral blood, it was counted microscopically after usual smear staining. The real number of each subset ($/mm^3$) was calculated from the number of peripheral lymphocytes multiplied by each subset in percentage obtained upon the flow cytometry. In the normal control group, the measurement was performed 40 times with 40 cases, and in the PD group it was performed a total of 50 times with 20 cases. In the stable or improved group, it was performed a total of 93 times with 24 cases. The results of the respective lymphocyte and monocyte counts are shown in Table 1.

Table 1

| Unit:/mm$^3$ | Normal control group (n=40) | Progressive disease group (n=50) | Stable or improved group (n=93) |
|---|---|---|---|
| OKT 3 | 1341*±413 | 742±454 | 880±407 |
| OKT 4 | 853±295 | 495±324 | 551±275 |
| OKT 8 | 606±239 | 315±235 | 377±194 |
| OKIal | 588±310 | 300±268 | 416±274 |
| Th | 423±155 | 311±222 | 346±182 |
| Tsi | 387±170 | 169±147 | 192±130 |
| Tc | 424±180 | 237±176 | 288±167 |
| Ts | 123±92 | 45±57 | 59±44 |
| Act.si/hT | 26.8±19.5 | 18.4±17.7 | 32.2±21.3 |
| Act.s/c T | 19.5±17.7 | 12.8±13.7 | 42.7±40.1 |
| NK cell | 201±106 | 95±75 | 160±117 |
| Monocyte | 330±142 | 396±260 | 396±243 |
| Act. T | 46.2±35.4 | 31.2±28.5 | 74.9±56.0 |
| Act. B | 542±294 | 269±265 | 342±265 |

* Mean ± SD

MULTIPLE REGRESSION ANALYSIS OF MONOCYTES AND EACH LYMPHOCYTE SUBSET IN THE PERIPHERAL BLOOD

For the purpose of knowing the mutual relationship between each lymphocyte subset and monocytes in the peripheral blood, multiple regression analysis was performed.

The multiple regression analysis was conducted to see whether or not the multiple regression equation is established in the respective groups (i.e. the normal control, progressive disease, and stable or improved groups), using Act.s/c T as dependent variables and monocytes and each peripheral lymphocyte subset as independent variables. Significant difference of partial regression coefficients was checked by t-test, and that of multiple correlation coefficients by f-test. Further, significant difference of simple correlation coefficients between subpopulations was examined by t-test.

MULTIPLE REGRESSION ANALYSIS USING Act.s/c T AS DEPENDENT VARIABLES AND EACH LYMPHOCYTE SUBSET AND MONOCYTES AS INDEPENDENT VARIABLES

a. NORMAL CONTROL GROUP

In the matrix of simple correlation coefficient Act. s/c T showed a correlation of as strong as $r=0.807$ ($p<0.01$) with Act.si/h T. Further, natural killer cells showed no correlation with either of them, and monocytes were related to Th ($r=0.246$), and to Act.si/h T ($r=0.344$, $p<0.05$).

The multiple regression equation was significantly established for Act.s/c T ($r=0.92893$, $p<0.01$; constant: 0.27972). It was also established significantly in terms of partial regression coefficient with the subsets Leu11+ ($p<0.05$), Tc ($p<0.05$), Ts ($p<0.05$) and Act.si/h T ($p<0.01$). The contributions were noted to be 0.83, 0.09, 3.73, 7.71, 65.05, 1.79 and 1.55% for Th, Tsi, Tc, Ts, Act.si/h T, monocytes and natural killer cells, respectively, and the total contribution was 86.29%. These results are shown in Table 2.

EP 0 443 036 B1

Table 2

(n=40)                                                                                                    (Dependent variable=Act. s/c T)

| Independent variables | OKT3 | OKT4 | OKT8 | OKIal | NK cell | Th | Tsi | Tc | Ts | Act. si/hT | Act. s/c T | Mono-cyte | Partial regression coefficient | Contribution (%) |
|---|---|---|---|---|---|---|---|---|---|---|---|---|---|---|
| OKT3 | | 0.835** | 0.802** | 0.625** | 0.230 | 0.783** | 0.529** | 0.727** | 0.497** | 0.380* | 0.331* | 0.132 | 0.00649 | 0.10 |
| OKT4 | 0.835** | | 0.427** | 0.441** | 0.287 | 0.682** | 0.809** | 0.361* | 0.268 | 0.339* | 0.109 | 0.08 | -0.03621* | 4.73 |
| OKT8 | 0.802** | 0.427** | | 0.683** | 0.221 | 0.593** | 0.129 | 0.837** | 0.661** | 0.348* | 0.473** | 0.240 | -0.02342 | 0.61 |
| OKIal | 0.625** | 0.441** | 0.683* | | 0.182 | 0.449** | 0.242 | 0.512** | 0.597** | 0.472** | 0.460** | 0.309 | -0.00026 | 0.00 |
| NK cell | 0.230 | 0.287 | 0.221 | 0.182 | | 0.072 | 0.156 | 0.017 | 0.227 | 0.162 | 0.218 | 0.297 | 0.03956* | 1.55 |
| Th | 0.783** | 0.682** | 0.593** | 0.449** | 0.072 | | 0.346* | 0.543** | 0.271 | 0.418** | 0.317* | 0.246 | 0.01838 | 0.83 |
| Tsi | 0.529** | 0.809** | 0.129 | 0.242 | 0.156 | 0.346* | | 0.092 | 0.196 | 0.208 | -0.005 | -0.087 | 0.00799 | 0.09 |
| Tc | 0.727** | 0.361* | 0.837** | 0.512** | 0.017 | 0.543** | 0.092 | | 0.372* | 0.130 | 0.310 | 0.095 | 0.03714* | 3.73 |
| Ts | 0.497** | 0.268 | 0.661** | 0.597** | 0.227 | 0.271 | 0.196 | 0.372* | | 0.425** | 0.596** | 0.100 | 0.05911* | 7.71 |
| Act. si/hT | 0.380* | 0.339* | 0.348* | 0.472** | 0.162 | 0.418** | 0.208 | 0.130 | 0.425** | | 0.807** | 0.344* | 0.73339** | 65.05 |
| Act. s/c T | 0.331* | 0.109 | 0.473** | 0.460** | 0.218 | 0.317* | -0.005 | 0.310 | 0.596** | 0.807** | | 0.204 | — | — |
| Monocyte | 0.132 | 0.080 | 0.240 | 0.309 | 0.297 | 0.246 | -0.087 | 0.095 | 0.100 | 0.344* | 0.204 | | -0.01738 | 1.79 |
| Constant | | | | | | | | | | | | | 0.27972 | 86.29 (Sum) |

Multiple correlation coefficient=0.92893 (p<0.01)          * p<0.05          **p<0.01   (t test)

13

b. PROGRESSIVE DISEASE GROUP

In the matrix of simple correlation coefficient Act.s/c T was correlated with Act.si/h T (r=0.633, p<0.01), while natural killer cells correlated with Tsi (r=0.818, p<0.01), Act.si/h T (r=0.507, p<0.01) and monocytes (r=0.285, p<0.05), respectively. Monocytes were correlated with Th (r=0.363, p<0.01), and Act.si/h T (r=0.109).

Act.s/c T, at r=0.80687 (p<0.01) and constant = 6.45252, showed establishment of the multiple regression equation with significant partial regression coefficients of OKT8 (p<0.05), and Act.si/h T (p<0.01), respectively. The contributions were 1.03, 0.29, 4.17, 13.25, 40.01, 3.82 and 0.14% for Th, Tsi, Tc, Ts, Act.si/h T, monocytes and natural killer cells, respectively and the total contribution was 65.11%. These results are shown in Table 3.

Table 3

(n=50)                                                                                                    (Dependent variables=Act. s/c T)

| Independent variables | OKT3 | OKT4 | OKT8 | OKIal | NK cells | Th | Tsi | Tc | Ts | Act. si/hT | Act. s/c T | Mono-cytes | Partial regression coefficient | Contribution (%) |
|---|---|---|---|---|---|---|---|---|---|---|---|---|---|---|
| OKT3 | | 0.966** | 0.849** | 0.792** | 0.499** | 0.88** | 0.779** | 0.839** | 0.564** | 0.308* | 0.307* | 0.38** | -0.01184 | 0.09 |
| OKT4 | 0.966** | | 0.753** | 0.824** | 0.409** | 0.901** | 0.843** | 0.761** | 0.452** | 0.276 | 0.23 | 0.406** | 0.0637 | 0.10 |
| OKT8 | 0.849** | 0.753** | | 0.544** | 0.645** | 0.626** | 0.497** | 0.947** | 0.768** | 0.243 | 0.43** | 0.322* | 0.01161* | 0.7 |
| OKIal | 0.792** | 0.824** | 0.544** | | 0.254 | 0.858** | 0.681** | 0.576** | 0.185 | 0.194 | 0.126 | 0.406** | 0.01458 | 1.51 |
| NK cells | 0.499** | 0.409** | 0.645** | 0.254 | | 0.253 | 0.264 | 0.575** | 0.818** | 0.507** | 0.574** | 0.285* | -0.00824 | 0.14 |
| Th | 0.88** | 0.901** | 0.626** | 0.858** | 0.253 | | 0.631** | 0.621** | 0.321* | 0.234 | 0.147 | 0.363** | 0.01279 | 1.03 |
| Tsi | 0.779** | 0.843** | 0.497** | 0.681** | 0.264 | 0.631** | | 0.563** | 0.194 | 0.284* | 0.167 | 0.266 | -0.05743 | 0.29 |
| Tc | 0.839** | 0.761** | 0.947** | 0.576** | 0.575** | 0.621** | 0.563** | | 0.665** | 0.185 | 0.296* | 0.337* | 0.05217 | 4.17 |
| Ts | 0.564** | 0.452** | 0.768** | 0.185 | 0.818** | 0.321* | 0.194 | 0.665** | | 0.436** | 0.603** | 0.156 | 0.05915 | 13.25 |
| Act. si/hT | 0.308* | 0.276 | 0.243 | 0.194 | 0.507** | 0.234 | 0.284* | 0.185 | 0.436** | | 0.633** | 0.109 | 0.34678** | 40.01 |
| Act. s/c T | 0.307* | 0.23 | 0.43** | 0.126 | 0.574** | 0.147 | 0.167 | 0.296* | 0.603** | 0.633** | | -0.08 | - | - |
| Monocyte | 0.38* | 0.406** | 0.322* | 0.406** | 0.285* | 0.363** | 0.266 | 0.337* | 0.156 | 0.109 | -0.08 | | -0.01104 | 3.82 |
| Constant | | | | | | | | | | | | | 6.45242 | 65.11 (Sum) |

Multiple correlation coefficient=0.80687 (p<0.01)          * p<0.05          **p<0.01  (t test)

c. STABLE OR IMPROVED GROUP

In the matrix of simple correlation coefficient Act.s/c T was correlated with Act.si/h T ($r=0.628$, $p<0.01$), while natural killer cells were correlated with Tsi ($r=-0.114$), Ts ($r=0.483$, $p<0.01$), Act.si/h T ($r=0.4$, $p<0.01$) and monocytes ($r=0.096$), respectively. Monocytes were correlated with Th ($r=0.283$, $p<0.01$), and Act.si/h T ($r=0.209$), respectively.

Act.s/c T, at $r=0.75889$ ($p<0.01$) and constant $= 3.15737$, showed establishment of the multiple regression equation with significant partial regression coefficients of OKT4 ($p<0.05$), natural killer cells ($p<0.05$) and Act.si/h T ($p<0.01$), respectively. The contributions were 0.31, 0.73, 3.9, 1.2, 39.45, 0.43 and 10.15% for Th, Tsi, Tc, Ts, Act.si/h T, monocytes and natural killer cells, respectively and the total contribution was 57.6%. These results are shown in Table 4.

Table 4

EP 0 443 036 B1

(n=93)         (Dependent variable=Act. s/c T)

| Independent variables | OKT3 | OKT4 | OKT8 | OKIal | NK cells | Th | Tsi | Tc | Ts | Act. si/hT | Act. s/c T | Mono-cytes | Partial regression coefficient | Contribution (%) |
|---|---|---|---|---|---|---|---|---|---|---|---|---|---|---|
| OKT3 | | 0.923** | 0.828** | 0.637** | 0.149 | 0.856** | 0.581** | 0.83** | 0.428** | 0.174 | 0.102 | 0.284** | 0.02189 | 0.15 |
| OKT4 | 0.923** | | 0.618** | 0.458** | 0.007 | 0.843** | 0.703** | 0.642** | 0.377** | 0.133 | -0.038 | 0.258** | -0.09823* | 1.13 |
| OKT8 | 0.828** | 0.618** | | 0.727** | 0.395** | 0.626** | 0.285** | 0.87** | 0.486** | 0.24 * | 0.288** | 0.185 | -0.03283 | 0.15 |
| OKIal | 0.637** | 0.453** | 0.727** | | 0.449** | 0.544** | 0.218* | 0.688** | 0.473** | 0.142 | 0.291** | 0.239* | 0.00116 | 0 |
| NK cells | 0.149 | 0.007 | 0.395** | 0.449** | | 0.011 | -0.114 | 0.235* | 0.483** | 0.4 ** | 0.54 ** | 0.096 | 0.0878 * | 10.15 |
| Th | 0.856** | 0.843** | 0.626** | 0.544** | 0.011 | | 0.404** | 0.63 ** | 0.255** | 0.2 * | 0.05 | 0.283** | 0.04006 | 0.31 |
| Tsi | 0.581** | 0.703** | 0.285** | 0.218* | -0.114 | 0.404** | | 0.411** | 0.252* | -0.014 | -0.079 | 0.104 | -0.03707 | 0.73 |
| Tc | 0.83 ** | 0.642** | 0.87 ** | 0.688** | 0.235* | 0.63 ** | 0.411** | | 0.347** | 0.129 | 0.221* | 0.272** | 0.06675 | 3.9 |
| Ts | 0.428** | 0.377** | 0.486** | 0.473** | 0.483** | 0.255** | 0.252* | 0.347** | | 0.446** | 0.435** | 0.185 | 0.16116 | 1.2 |
| Act. si/hT | 0.174 | 0.133 | 0.24 * | 0.142 | 0.4 ** | 0.2 * | -0.014 | 0.129 | 0.446** | | 0.628** | 0.029 | 0.87656** | 39.45 |
| Act. s/c T | 0.102 | -0.038 | 0.288** | 0.291** | 0.543** | 0.05 | -0.079 | 0.221* | 0.435** | 0.628** | | -0.013 | — | — |
| Monocytes | 0.284** | 0.258** | 0.185 | 0.239* | 0.096 | 0.283** | 0.104 | 0.272** | 0.185 | 0.029 | -0.013 | | -0.01412 | 0.43 |
| Constant | | | | | | | | | | | | | 3.15737 | 57.60 (Sum) |

Multiple correlation coefficient=0.75889 (p<0.01)      * p<0.05     **p<0.01 (t test)

MULTIPLE REGRESSION ANALYSIS USING MONOCYTE AS DEPENDENT VARIABLES AND Act.si/h T AND Th AS INDEPENDENT VARIABLES

a. NORMAL CONTROL GROUP

In the normal control group monocytes singly established a relation with Act.si/h T at r = 0.34396 (p<0.05). It means that the autologous tumor antigen recognition mechanism (ATARM) from monocytes to Mø and Act.Th is established. Further, it is one of the criteria for determination in comparison with other groups that correlation is at about r=0.34396 in the normal.

b. PROGRESSIVE DISEASE GROUP

Monocytes showed a multiple correlation with Th and Act.si/h T at r = 0.36376 (p<0.05). However, the contribution of Act.si/h T was extremely reduced (to as low as 0.06%) as compared with the normal, and they were mainly related to Th, a precursor cell of Act.Th, it thus being shown that the ATARM was reduced extremely.

c. STABLE OR IMPROVED GROUP

As in the progressive disease group, monocytes showed a multiple correlation at r=0.2486 (p<0.05), but the contribution of Act.si/h T was as low as 0.08% and the ATARM was reduced extremely. These results are shown in Table 5.

Table 5

| Dependent variables =Monocytes | Normal control group (n=40) | | Progressive disease group (n=50) | | Stable or improved group (n=93) | |
|---|---|---|---|---|---|---|
| Independent variables | Partial regression coefficient | Contribution (%) | Partial regression coefficient | Contribution (%) | Partial regression coefficient | Contribution (%) |
| Th | − | − | 0.41948* | 13.17 | 0.38468** | 8.02 |
| Act. si/hT | 2.4962* | 11.83 | 0.37159 | 0.06 | -0.32469 | 0.08 |
| Constant | 263.222 | Sum 11.83 | 258.697 | Sum 13.23 | 273.056 | Sum 8.10 |
| Multiple correlation coefficient | =0.34396(p<0.05) | | =0.36376(p<0.05) | | =0.2846 (p<0.05) | |

*p<0.05    **p<0.01    (t test)

MULTIPLE REGRESSION ANALYSIS USING NATURAL KILLER CELLS AS DEPENDENT VARIABLES AND Act.Si/h T AND Tsi AS INDEPENDENT VARIABLES

a. NORMAL CONTROL GROUP

NK cell did not establish a relation with Act.si/h T and Tsi, which means that the activity of natural killer cells has not been potentiated.

b. PROGRESSIVE DISEASE GROUP

Natural killer cells established a relation only with Act.si/h T at r=0.50688 (p<0.01). This means a strong relationship with Act.Tsi, suggesting an intensified activation by the HLBI treatment.

c. STABLE OR IMPROVED GROUP

As in the progressive disease group, natural killer cells established a relation only with Act.si/h T at r=0.39953 (p<0.01), suggesting an intensified activation.
These results are shown in Table 6.

Table 6

| Dependent variable =NK cell | Normal control group (n=40) | | Progressive disease group (n=50) | | Stable or improved group (n=93) | |
|---|---|---|---|---|---|---|
| Independent variables | Partial regression coefficient | | Partial regression coefficient | | Partial regression coefficient | |
| | | Contribution (%) | | Contribution (%) | | Contribution (%) |
| Tsi | 0.08025 | 1.58 | -. | – | – | – |
| Act. si/hT | 0.73532 | 2.62 | 2.15649** | 25.69 | 2.1898** | 15.96 |
| Constant | 149.825 | Sum 4.20 | 55.4017 | Sum 25.69 | 89.4474 | Sum 15.96 |

Multiple correlation coefficient    =0.20490(n.s.)    =0.50688(p<0.01)    =0.39953(p<0.01)

n.s.: not significant    *p<0.05    **p<0.01    (t test)

## MULTIPLE REGRESSION ANALYSIS USING OKIa1 ANTIGEN POSITIVE LYMPHOCYTES AS DEPENDENT VARIABLES AND MONOCYTES AND NATURAL KILLER CELLS AS INDEPENDENT VARIABLES

Since lymphocytes and monocytes are considered to be activated upon expression of the major histocompatibility complex: Ia antigen or HLA-DR antigen, the multiple regression analysis was conducted to study contribution of the monocytes and the natural killer cells related to expression of Ia antigen using the OKIa1 antigen positive lymphocytes count as dependent variable.

### a. NORMAL CONTROL GROUP

The multiple regression was not established being insignificant at r=0.32308. It means that in the normal human at r=0.32308, a contribution of the monocytes 9.54% and that of the NK cells 0.89%, is maintained a homeostasis, thus immunologically monocytes and natural killer cells are participating neither in activation nor in suppression of expression of Ia antigen. Such multiple correlation coefficient and contributions are the standard values to estimate whether monocytes and natural killer cells have been activated or suppressed.

### b. PROGRESSIVE DISEASE GROUP

The expression of Ia antigen was dependent significantly on monocytes compared with the normal, and the contribution of natural killer cells was also higher, which is considered to be due to the α-IFN treatment.

c. STABLE OR IMPROVED GROUP

The contributions and multiple correlation coefficient were even higher, showing a stronger dependence on monocytes and natural killer cells. This is considered to be due to the $\alpha$-IFN treatment, etc., but functions of monocytes and NK cells were more activated than in the normal.

Those results are shown in Table 7.

Table 7

| Dependent variables =OKIa1 | Normal control group (n=40) | | Progressive disease group (n=50) | | Stable or improved group (n=93) | |
|---|---|---|---|---|---|---|
| Independent variables | Partial regression coefficient | Contribution (%) | Partial regression coefficient | Contribution (%) | Partial regression coefficient | Contribution (%) |
| Monocytes | 0.61131 | 9.54 | 0.37391** | 16.47 | 0.22353* | 20.19 |
| NK cells | 0.289 | 0.89 | 0.53554 | 2.09 | 1.01107** | 3.87 |
| Constant | 328.051 | Sum 10.43 | 100.99 | Sum 18.56 | 166.253 | Sum 24.06 |

Multiple correlation coefficient      =0.32308(n.s.)     =0.43076(p<0.01)     =0.4906(p<0.01)

n.s.: not significant      *p<0.05   **p<0.01   (t test)

The foregoing results confirm that, in the normal control group which is not cancer-bearing, neither activation nor suppression takes place and no activities of monocytes and natural killer cells related to expression of Ia antigen are recognized.

However, in the PD group and the stable or improved group, where influence of $\alpha$-IFN is considered to be great, it was demonstrated that compared with the normal control group functions of monocytes and natural killer cells were significantly accelerated to activate lymphocytes by expression of Ia antigen. It has also been indicated in specific figures that in the PD group too functions of monocytes and NK cells are more accelerated compared with the normal, but that it is impossible to improve the disease with the multiple correlation coefficient or contributions as shown in Table 7. At least to stabilize or improve the disease, as noted in the results of multiple regression analysis for the stable or improved group in Table 7 (contribution of monocytes 20.19% and that of natural killer cells 3.87%), it can be understood that compared with the normal control, an activation a little over 2 times for monocytes and that a little over 4 times for natural killer cells, are required.

Thus, according to the immunokinetic measurement of this invention, namely by performing any of the above-mentioned procedures (A)-(C) to determine the mutual relationship between each factor as dependent variable and the respective factors as independent variables, it is possible to estimate to which one of the groups (i.e. the normal control, progressive disease and stable or improved groups) the result of measurement with a patient under therapy belongs, and to determine whether the treatment is appropriate or not.

For the purpose of knowledge of more detailed immunokinetics, cellular immunokinematograms should be drawn.

## CELLULAR IMMUNOKINEMATOGRAM

For making kinematograms of cellular immunity, contributions of each subset and monocytes obtained by the multiple regression analysis using Act.s/c T as dependent variable were arranged directly as areas of the circle based on the mutual relationship and process of differentiation and maturation of each subset generally established nowadays basically and clinically. Meanwhile, it has not been clarified specifically yet up to date to which subset the natural killer cells are related most strongly, and what its major activity is. Therefore, based on the simple correlation coefficient matrix of the analytical results obtained in this study, it was supposed to have a mutual relationship with the lymphocytes showing the strongest correlation. For these reasons, in practice, contribution of Act.si/h T was partitioned according to those of the respective precursor cells, Tsi and Th, to calculate contributions of Act.Tsi and Act.Th. In the same manner the total contribution of Act.s/c T was divided in proportion to the ratio of Ts and Tc, to calculate contributions of Act.Ts and Act.Tc.

Further, in the case of Mø, the contribution of monocytes as such was used as that of Mø.

The contributions of Act.Th, Act.Tc, Act.Ts and Act.Tsi were calculated as follows. Act.Th in the normal control is determined by [Th/(Th + Tsi)] x Act.si/h T. The contribution of each subset from Table 2 is substituted for the corresponding term to obtain [0.83 ÷ (0.83 + 0.09)] x 65.05 = 58.69 (%). In the same way, Act.Tc is [Tc/(Tc + Ts)] x Act.s/c T, and substitution of values gives [3.73 ÷ (3.73 + 7.71)] x 86.29 = 28.13 (%). Thus the contributions of Act.Tsi and Act.Ts were calculated and expressed as areas of circle, and the circular areas were arranged in accordance with the mutual relations between different peripheral lymphocyte subsets to obtain cellular kinematograms, as shown in Fig. 1.

Immunokinetics between the groups can be grasped at a glance from such cellular immunokinematograms. Such kinematograms make it possible to better understand behaviors of immunologically competent cell in each group, thus being shown to be useful in treatment including evaluation of the therapeutic effect. Thus, from determinations of peripheral lymphocyte subsets are obtainable practical and easy-to-understand kinematograms, which make it possible to reveal a series of cellular immunokinetics following the ATARM.

## Example 2

Measurement of Cellular Immunokinetics Based upon Autologous Tumor Antigen Recognition Mechanism in Easily-Recurrent Superficial Bladder Cancer Patients.

## MATERIALS

The subjects in this study consisted of 59 such patients who were admitted and treated in the Department of Urology, Cancer Institute Hospital between February 1977 and December 1985. Of 59 patients, 27 experienced recurrence less than three years after the previous transurethral resection of tumor (TUR), and peripheral lymphocyte subsets were determined when such recurrence was confirmed. The remaining 32 including nine patients with the history of recurrence had experienced no recurrence for three years or more after the initial TUR or treatment of recurrent cancer. Peripheral lymphocyte subsets were determined in these cases at the final observation. The former and latter patients were classified as the recurrent and not-recurrent groups, respectively and the results of determination were analyzed. As measured values for the normal control group were adopted those obtained with the normal control group in Example 1.

## DETERMINATION OF PERIPHERAL LYMPHOCYTE SUBPOPULATIONS AND MONOCYTES

The same procedure as in Example 1 was followed to obtain the values as shown in Table 8.

Table 8

| Unit:/mm$^3$ | Normal control group (n=40) | | Recurrent group (n=27) | | Not-recurrent group (n=32) | |
|---|---|---|---|---|---|---|
| OKT 3 | 1341 | ± 413 | 1349 | ± 353 | 1506 | ± 600 |
| OKT 4 | 853 | ± 295 | 894 | ± 348 | 874 | ± 358 |
| OKT 8 | 606 | ± 239 | 624 | ± 294 | 750 | ± 346 |
| OKIal | 588 | ± 310 | 470 | ± 207 | 544 | ± 380 |
| Th | 423 | ± 155 | 505 | ± 237 | 480 | ± 247 |
| Tsi | 387 | ± 170 | 352 | ± 183 | 367 | ± 217 |
| Tc | 424 | ± 180 | 468 | ± 283 | 607 | ± 382 |
| Ts | 123 | ± 92 | 101 | ± 68 | 144 | ± 86 |
| Act.si/hT | 26.8 | ± 19.5 | 46.6 | ± 38.9 | 52.8 | ± 40.6 |
| Act.s/c T | 19.5 | ± 17.7 | 35.0 | ± 34.4 | 59.4 | ± 61.0 |
| NK cells | 201 | ± 106 | 187 | ± 163 | 228 | ± 177 |
| Monocytes | 330 | ± 142 | 337 | ± 188 | 378 | ± 234 |
| Lymphocytes | 2100 | ± 585 | 2237 | ± 707 | 2466 | ± 811 |

Mean ± SD

MULTIPLE REGRESSION ANALYSIS OF MONOCYTES AND EACH PERIPHERAL LYMPHOCYTE SUBPOPULATION

In the same manner as in Example 1, the multiple regression analysis was performed for each group (i.e. the normal control, recurrent, and not-recurrent groups), using Act.s/c T as dependent variables and monocytes and subpopulations, as independent variables to determine whether the multiple regression equation was established or not. The significance of differences in the partial regression coefficients was assessed by t-test and that of differences in the multiple correlation coefficient, by f-test. Further, the significance of subpopulation-related differences in the simple correlation coefficient was examined by t-test.

Further, as in Example 1, multiple regression analysis where monocytes were used as dependent variables and Th and Act.si/h T as independent variables was performed for the purpose of investigating the ATARM. Furthermore multiple regression analysis where natural killer cells were used as dependent variables and Tsi and Act.si/h T as independent variables was performed to evaluate the function of natural killer cells. In addition multiple regression analysis where OKIa1 antigen positive lymphocytes were used as dependent variable and monocytes and natural killer cells as independent variables was performed for the purpose of investigating cooperation between monocytes and natural killer cells within or around cancer tissues.

MULTIPLE REGRESSION ANALYSIS WITH Act.s/c T AS DEPENDENT VARIABLES AND OTHER PERIPHERAL LYMPHOCYTE SUBPOPULATIONS AND MONOCYTES AS INDEPENDENT VARIABLES

a. NORMAL CONTROL GROUP

In the matrix of simple correlation coefficient, Act.s/c T was noted to correlate strongly with Act.si/h T ($r=0.807$, $p<0.01$). Further, Leu $11^+$ (natural killer cells) was not correlated with any subset and monocytes were related to Th ($r=0.246$) and Act.si/h T ($r=0.344$, $p<0.05$).

The multiple regression equation was significantly established for Act.s/c T ($r=0.92893$, $p<0.01$; constant $= 0.27972$). It was also established significantly in terms of partial regression coefficient with natural killer cells ($p<0.05$), Tc ($p<0.05$), Ts ($p<0.05$) and Act.si/h T ($p<0.01$). The contributions were 0.83, 0.09, 3.73, 7.71, 65.05, 1.79 and 1.55% for Th, Tsi, Tc, Ts, Act.si/h T, monocytes and natural killer cells, respectively and the total contribution was 86.29%. The results are shown in Table 9.

Table 9

(n=40)

(Dependent variables=Act. s/c T)

| Independent variables | OKT3 | OKT4 | OKT8 | OKIal | NK cells | Th | Tsi | Tc | Ts | Act. si/hT | Act. s/c T | Mono-cytes | Partial regression coefficient | Contribution (%) |
|---|---|---|---|---|---|---|---|---|---|---|---|---|---|---|
| OKT3 | | 0.835** | 0.802** | 0.625** | 0.230 | 0.783** | 0.529** | 0.727** | 0.497** | 0.380* | 0.331* | 0.132 | 0.00649 | 0.10 |
| OKT4 | 0.835** | | 0.427** | 0.441** | 0.287 | 0.682** | 0.809** | 0.361* | 0.268 | 0.339* | 0.109 | 0.08 | -0.03621* | 4.73 |
| OKT8 | 0.802** | 0.427** | | 0.683** | 0.221 | 0.593** | 0.129 | 0.837** | 0.661** | 0.348* | 0.473** | 0.240 | -0.02342 | 0.61 |
| OKIal | 0.625** | 0.441** | 0.683** | | 0.182 | 0.449** | 0.242 | 0.512** | 0.597** | 0.472** | 0.460** | 0.309 | 0.00026 | 0.00 |
| NK cells | 0.230 | 0.287 | 0.221 | 0.182 | | 0.072 | 0.156 | 0.017 | 0.227 | 0.162 | 0.218 | 0.297 | 0.03956* | 1.55 |
| Th | 0.783** | 0.682** | 0.593** | 0.449** | 0.072 | | 0.346* | 0.543** | 0.271 | 0.418** | 0.317* | 0.246 | 0.01838 | 0.83 |
| Tsi | 0.529** | 0.809** | 0.129 | 0.242 | 0.156 | 0.346* | | 0.092 | 0.196 | 0.208 | -0.005 | -0.087 | 0.00799 | 0.09 |
| Tc | 0.727* | 0.361* | 0.837** | 0.512** | 0.017 | 0.543** | 0.092 | | 0.372* | 0.130 | 0.310 | 0.095 | 0.03714* | 3.73 |
| Ts | 0.497** | 0.268 | 0.661** | 0.597** | 0.227 | 0.271 | 0.196 | 0.372* | | 0.425** | 0.596** | 0.100 | 0.05911* | 7.71 |
| Act. si/hT | 0.380* | 0.339* | 0.348* | 0.472** | 0.162 | 0.418** | 0.208 | 0.130 | 0.425** | | 0.807** | 0.344* | 0.73339** | 65.05 |
| Act. s/c T | 0.331* | 0.109 | 0.473** | 0.460** | 0.218 | 0.317* | -0.005 | 0.310 | 0.596** | 0.807** | | 0.204 | - | - |
| Monocytes | 0.132 | 0.080 | 0.240 | 0.309 | 0.297 | 0.246 | -0.087 | 0.095 | 0.100 | 0.344* | 0.204 | | -0.01738 | 1.79 |
| Constant | | | | | | | | | | | | | 0.27972 | 86.29 (Sum) |

Multiple correlation coefficient=0.92893 (p<0.01)     * p<0.05     **p<0.01 (t test)

b. RECURRENT GROUP

In the matrix of simple correlation coefficient, Act.s/c T was noted to correlate with Act.si/h T ($r=0.886$, $p<0.01$). Natural killer cells were related to Tsi ($r=0.465$, $p<0.05$), Ts ($r=0.502$, $p<0.01$), Act.si/h T ($r=0.586$, $p<0.01$), Act.s/c T ($r=0.590$, $p<0.01$) and monocytes ($r=0.016$). Monocytes were related to Th ($r=0.039$) and Act.si/h T ($r=0.213$).

The multiple regression equation was significantly established for Act.s/c T ($r=0.94152$, $p<0.01$; constant: -2.74151). It was also significantly established only with Act.si/h T ($p<0.01$) in terms of partial regression coefficient. The contributions were 2.38, 0, 0.17, 0.22, 78.56, 2.16, and 0.14% for Th, Tsi, Tc, Ts, Act.si/h T, monocytes and natural killer cells, respectively and the total contribution was 88.63%. These results are shown in Table 10.

Table 10

(Dependent variable: Act. s/c T)

(n=27)

| Independent variables | OKT3 | OKT4 | OKT8 | OKIa1 | NK cells | Th | Ts1 | Tc | Ts | Act. si/hT | Act. s/c T | Monocytes | Partial regression coefficient | Contribution (%) |
|---|---|---|---|---|---|---|---|---|---|---|---|---|---|---|
| OKT3 | | 0.617** | 0.620** | 0.670** | 0.412* | 0.575** | 0.443* | 0.532** | 0.111 | 0.364 | 0.379 | 0.232 | 0.00035 | 0.00 |
| OKT4 | | | 0.342 | 0.335 | 0.640** | 0.471* | 0.583** | 0.235 | 0.350 | 0.617** | 0.547** | 0.276 | -0.00870 | 0.11 |
| OKT8 | | | | 0.522** | 0.252 | 0.314 | 0.093 | 0.864** | 0.503** | 0.069 | 0.617** | 0.202 | 0.01227 | 4.89 |
| OKIa1 | | | | | 0.099 | 0.240 | 0.241 | 0.595** | -0.114 | -0.013 | 0.090 | -0.013 | 0.00182 | 0.00 |
| NK cells | | | | | | 0.378 | 0.465* | 0.046 | 0.502 | 0.586** | 0.590** | 0.016 | 0.01071 | 0.14 |
| Th | | | | | | | 0.187 | 0.182 | 0.116 | 0.368 | 0.269 | 0.039 | -0.02003 | 2.38 |
| Ts1 | | | | | | | | 0.025 | 0.246 | 0.473* | 0.432* | 0.071 | -0.00056 | 0.00 |
| Tc | | | | | | | | | 0.155 | -0.076 | 0.117 | 0.322 | 0.02063 | 0.17 |
| Ts | | | | | | | | | | 0.344 | 0.501** | -0.113 | 0.04733 | 0.22 |
| Act. si/hT | | | | | | | | | | | 0.886** | 0.213 | 0.85532** | 78.56 |
| Act. s/c T | | | | | | | | | | | | 0.090 | - | - |
| Monocytes | | | | | | | | | | | | | -0.02807 | 2.16 |
| Constant | | | | | | | | | | | | | -2.74151 | - |
| | | | | | | | | | | | | | | 88.63 (Sum) |

Multiple correlation coefficient=0.94152 (p<0.01)    * p<0.05    **p<0.01 (t test)

c. NOT-RECURRENT GROUP

In the matrix of simple correlation coefficient, Act.s/c T was noted to correlate with Act.si/h T ($r=0.754$, $p<0.01$) and natural killer cells were related to Tsi ($r=0.046$), Ts ($r=0.152$), Act.si/h T ($r=0.195$) and monocytes ($r=-0.071$). Monocytes were related to Th ($r=0.485$, $p<0.01$) and Act.si/h T ($r=-0.069$).

The multiple regression equation was significantly established for Act.s/c T ($r=0.87106$, $p<0.01$; constant: 0.84916). It was significantly established only with Act.si/h T ($p<0.01$) in terms of partial regression coefficient. The contributions were 1.5, 6.22, 3.87, 6.67, 56.81, 0.18, and 0.02% for Th, Tsi, Tc, Ts, Act.si/h T, monocytes and natural killer cells, respectively and the total contribution was 75.87%. These results are shown in Table 11.

Table 11

(n=32)  (Dependent variable=Act. s/c T)

| Independent variables | OKT3 | OKT4 | OKT8 | OKIal | NK cells | Th | Tsi | Tc | Ts | Act. si/hT | Act. s/c T | Mono-cytes | Partial regression coefficient | Contribution (%) |
|---|---|---|---|---|---|---|---|---|---|---|---|---|---|---|
| OKT3 |  | 0.858** | 0.854** | 0.635** | -0.063 | 0.700** | 0.621** | 0.857** | 0.022 | 0.021 | -0.055 | 0.497** | -0.02418 | 0.17 |
| OKT4 | 0.858** |  | 0.543** | 0.450** | -0.068 | 0.797** | 0.735** | 0.536** | -0.107 | 0.126 | -0.140 | 0.441* | 0.05282 | 0.06 |
| OKT8 | 0.854** | 0.543** |  | 0.702** | -0.056 | 0.468** | 0.318 | 0.937** | 0.064 | -0.123 | 0.020 | 0.573** | 0.04617 | 0.19 |
| OKIal | 0.635** | 0.450** | 0.702** |  | 0.355* | 0.371* | 0.308 | 0.627** | -0.013 | -0.059 | -0.058 | 0.420* | -0.01453 | 0.18 |
| NK cells | -0.063 | -0.068 | -0.056 | 0.355* |  | -0.076 | 0.046 | -0.111 | 0.152 | 0.195 | 0.124 | -0.071 | 0.00658 | 0.02 |
| Th | 0.700** | 0.797** | 0.468** | 0.371* | -0.076 |  | 0.301 | 0.431* | -0.166 | 0.299 | 0.054 | 0.485** | -0.06938 | 1.50 |
| Tsi | 0.621** | 0.735** | 0.318 | 0.308 | 0.046 | 0.301 |  | 0.359* | 0.187 | 0.081 | -0.187 | -0.053 | -0.12426 | 6.22 |
| Tc | 0.857** | 0.536** | 0.937** | 0.627** | -0.111 | 0.431* | 0.359* |  | 0.069 | -0.154 | 0.001 | 0.457** | 0.04564 | 3.87 |
| Ts | 0.022 | -0.107 | 0.064 | -0.013 | 0.152 | -0.166 | 0.187 | 0.069 |  | 0.557** | 0.596** | -0.260 | 0.10824 | 6.67 |
| Act. si/hT | 0.21 | 0.126 | -0.123 | -0.059 | 0.195 | 0.299 | 0.081 | -0.154 | 0.557** |  | 0.754** | -0.069 | 1.22341** | 56.81 |
| Act. s/c T | -0.055 | -0.140 | 0.020 | -0.058 | 0.124 | 0.054 | -0.187 | 0.001 | 0.596** | 0.754** |  | -0.034 | – | — |
| Monocyte | 0.497** | 0.441* | 0.573** | 0.420* | -0.071 | 0.485** | -0.053 | 0.457** | -0.260 | -0.069 | -0.034 |  | -0.02212 | 0.18 |
| Constant |  |  |  |  |  |  |  |  |  |  |  |  | 0.84196 | 75.87 (Sum) |

Multiple correlation coefficient=0.87106 (p<0.01)　　　* p<0.05　　**p<0.01　(t test)

EP 0 443 036 B1

29

MULTIPLE REGRESSION ANALYSIS WITH MONOCYTES AS DEPENDENT VARIABLES AND Act.si/h T AND Th AS INDEPENDENT VARIABLES

a. NORMAL CONTROL GROUP

Monocytes were related to Act.si/h T (r=0.34396, p<0.05) and the equation of relation was established with such cells alone. This suggested that the ATARM from monocytes to Mø and to Act. Th was established. Further, a correlation coefficient (r) of about 0.34396 in normal persons may be used as a standard for evaluation in group comparison in the future.

b. RECURRENT GROUP

The multiple regression equation was not established between monocytes on the one hand and Th and Act.si/h T on the other (r=0.32754). In particular, the contribution rate of Act.si/h T was lower (4.55%) than in the normal control group (11.83%). It was indicated by this finding that the relationship between monocytes and helper-series T lymphocytes was extremely impaired. Since no significance was noted, it was considered completely impaired.

c. NOT-RECURRENT GROUP

In the not-recurrent group, the multiple regression equation was significantly established (r=0.53491, p<0.01) and recovery of function between monocytes and helper-series T lymphocytes was indicated. However, since Th as precursor cells accounted for the greater part in contribution and the value of partial regression coefficient for Act.si/h T was negative, predominance of Act.Tsi rather than Act. Th was suggested. Thus, it was suggested that the relationship between monocytes and helper-series T lymphocytes had not yet functioned normally. These results are shown in Table 12.

EP 0 443 036 B1

Table 12

| Dependent variable =Monocytes | Normal control group (n=40) | | Recurrent group (n=27) | | Not-recurrent group (n=32) | |
|---|---|---|---|---|---|---|
| Independent variables | Partial regression coefficient | Contribution (%) | Partial regression coefficient | Contribution (%) | Partial regression coefficient | Contribution (%) |
| Th | – | – | -0.03609 | 0.18 | 0.52569** | 23.56 |
| Act. si/hT | 2.4962* | 11.83 | 1.11382 | 4.55 | -1.35543 | 5.05 |
| Constant | 263.222 | Sum 11.83 | 303.305 | Sum 4.73 | 197.47 | Sum 28.61 |

Multiple correlation coefficient =0.34396(p<0.01)     =0.21754(n.s.)     =0.53491(p<0.01)

n.s.: not significant                *p<0.05        **p<0.01 (t test)

MULTIPLE REGRESSION ANALYSIS WITH NATURAL KILLER CELLS AS DEPENDENT VARIABLES AND Act.si/h T AND Tsi AS INDEPENDENT VARIABLES

a. NORMAL CONTROL GROUP

The multiple regression equation was not established with natural killer cells and it was suggested that these cells were not activated functionally.

b. RECURRENT GROUP

In the recurrent group, the equation of relation was established only with Act.si/h T (r=0.58626, p<0.01) and it was shown that natural killer cells were activated functionally.

c. NOT-RECURRENT GROUP

In the not-recurrent group, the multiple regression equation was not established and the function of natural killer cells was found almost the same as in the normal control group.

These results are shown in Table 13.

31

Table 13

| Dependent variable =NK cells | Normal control group (n=40) | | Recurrent group (n=27) | | Not-recurrent group (n=32) | |
|---|---|---|---|---|---|---|
| Independent variables | Partial regression coefficient | Contribution (%) | Partial regression coefficient | Contribution (%) | Partial regression coefficient | Contribution (%) |
| Tsi | 0.08025 | 1.58 | – | – | 0.02445 | 0.09 |
| Act. si/hT | 0.73532 | 2.62 | 2.45356** 34.37 | | 0.83908 | 3.81 |
| Constant | 149.825 | Sum 4.20 | 72.6671 | Sum 34.37 | 174.908 | Sum 3.90 |

Multiple correlation coefficient =0.20490(n.s.)   =0.58626(p<0.01)   =0.19737(n.s.)

n.s.:  not significant       *p<0.05     **p<0.01 (t test)

MULTIPLE REGRESSION ANALYSIS WITH OKIa1 ANTIGEN POSITIVE LYMPHOCYTES AS DEPENDENT VARIABLE AND MONOCYTES AND NATURAL KILLER CELLS AS INDEPENDENT VARIABLES

a. NORMAL CONTROL GROUP

In the normal control group, no significance was noted (r=0.32308) and the multiple regression equation was not established.

Since it was unnecessary in normal persons that OKIa1 antigen is developed to activate lymphocytes or monocytes, the contributions of 9.54 and 0.89% for monocytes and natural killer cells, respectively, were considered to represent normal conditions undergoing neither immunological enhancement nor suppression.

b. RECURRENT GROUP

In the recurrent groups, the multiple regression equation was not established (r=0.09982) and the contributions of monocytes and natural killer cells were extremely decreased. These results suggested hypofunction of both cells. In particular, the negative partial regression coefficient for monocytes suggested predominance of suppressor Mø.

c. NOT-RECURRENT GROUP

In the not-recurrent group, the multiple regression equation was significantly established (r=0.57028, p<0.01) and the contributions of monocytes and natural killer cells were increased. The results suggested that

32

their functions were activated. In particular, it was shown by the positive values of partial regression coefficient for both subsets that the normal cooperative relationship was maintained immunologically and activated Mø was predominant. These results are shown in Table 14.

Table 14

| Dependent variable =OKIal | Normal control group (n=40) | | Recurrent group (n=27) | | Not-recurrent group (n=32) | |
|---|---|---|---|---|---|---|
| Independent variables | Partial regression coefficient | Contribution (%) | Partial regression coefficient | Contribution (%) | Partial regression coefficient | Contribution (%) |
| Monocytes | 0.61131 | 9.54 | -0.01627 | 0.97 | 0.72737** | 17.64 |
| NK cells | 0.289 | 0.89 | 0.12556 | 0.02 | 0.83061* | 14.88 |
| Constant | 328.051 | Sum 10.43 | 452.36 | Sum 0.99 | 79.5165 | Sum 32.52 |

Multiple correlation coefficient   =0.32308(n.s.)    =0.09982(n.s.)    =0.57028(p<0.01)

n.s.: not significant       *p<0.05       **p<0.01 (t test)

It was confirmed by the foregoing results in the normal control group that the immunity promoting series based on the ATARM from monocytes to Mø and to Act.Th was almost balanced with the immunosuppressive series from Act. Tsi to Act.Ts, that Act.Tc maintained its function and also that the function of NK cells was neither potentiated nor suppressive on Act.Tsi.

In the recurrent group, the ATARM from monocytes to Act. Th was completely impaired. Natural killer cells were found to exert a significant suppressive action on Act.Tsi.

In the not-recurrent group, suppressive lymphocytes were predominant as compared with the normal control group, and the ATARM from monocytes to Act.Th was still impaired. A strong relationship was noted between helper T lymphocytes and monocytes but an inverse correlation between activated helper T lymphocytes and monocytes. This explains no significant suppressive function of natural killer cells on Act.Tsi.

Thus, as in Example 1, it is possible to estimate to which one of the groups (i.e. the normal control, recurrent and not-recurrent groups) the result of measurement with a patient under therapy belongs, and to determine whether the treatment is appropriate or not.

For the purpose of more detailed decision-making of treatment, cellular immunokinematograms should be drawn as follows.

CELLULAR IMMUNOKINEMATOGRAM

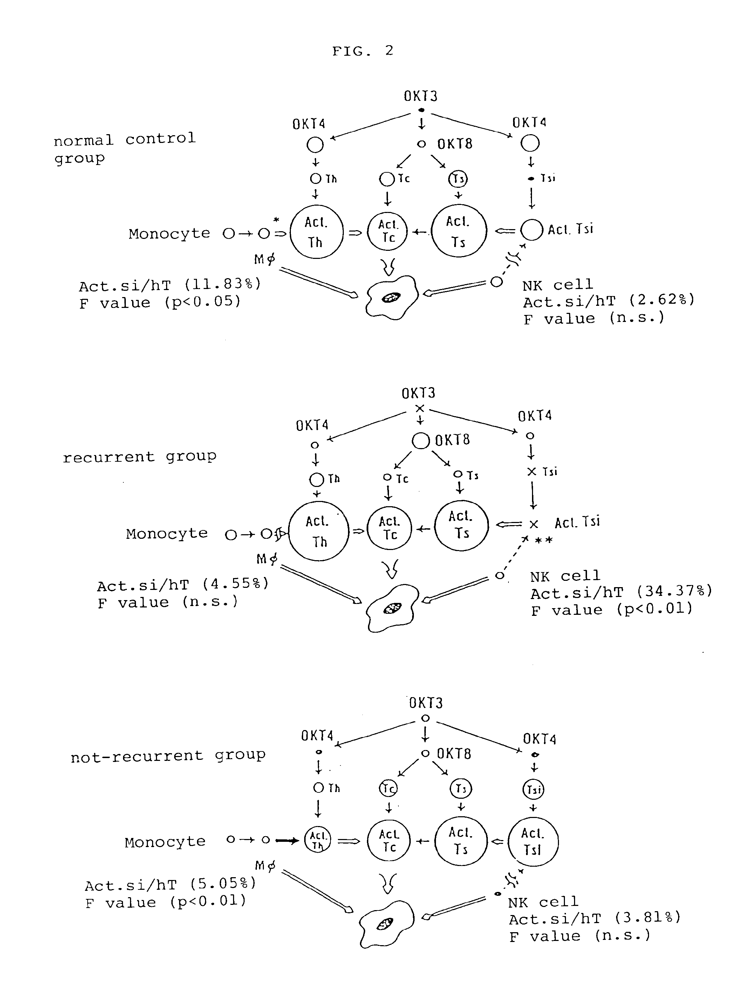

In drawing cellular immunokinematograms, the contributions obtained as a result of the multiple regression analysis were arranged on the basis of the mutual relationship between different subsets, in the same manner as in Example 1. The cellular immunokinematograms thus obtained are shown in Figure 2.

From these immunokinematograms can be shown the following:

In the recurrent group, helper- and suppressor-series T lymphocytes were seemingly well-balanced with each other to maintain the function of Act.Tc. However, the process from monocytes to Act.Th was impaired completely.

In the not-recurrent group, suppressor-series T lymphocytes were predominant. In the process from monocytes to Act.Th, however, suppressive Mø was predominant and its suppressive function was confirmed to be potentiated.

Example 3

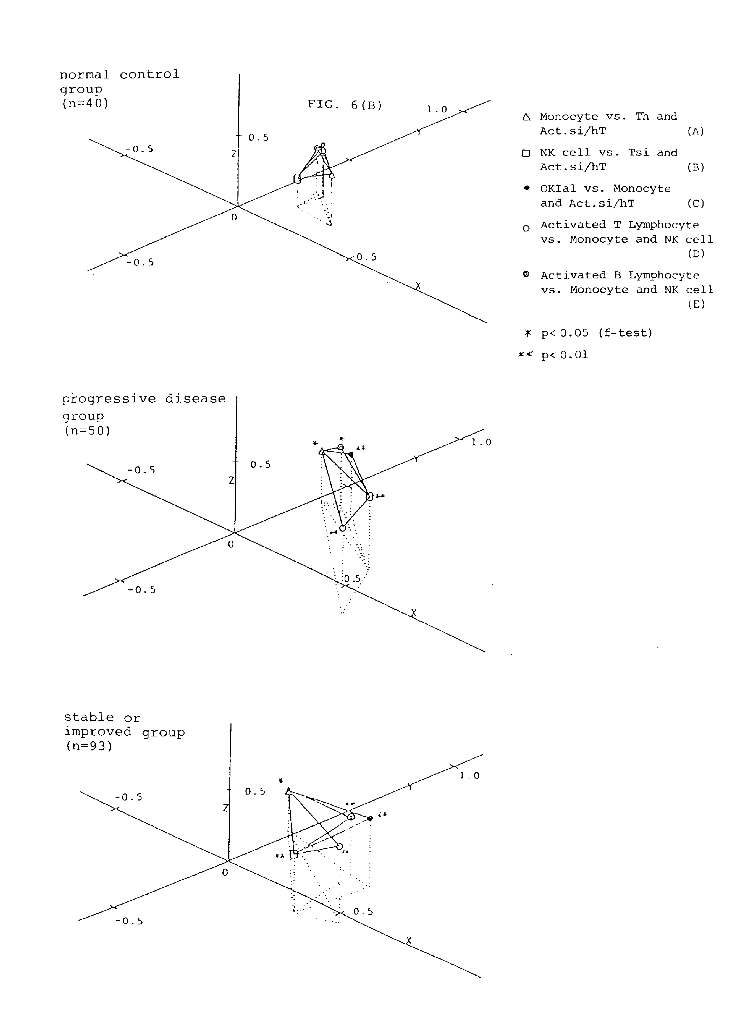

(1) The counts of each peripheral lymphocyte subpopulation and monocyte obtained in Example 1 were used to perform the above-mentioned multiple regression analyses (MRAs) (A) - (E), namely "the immunologic three-factor sets", i.e. MRAs with monocytes as dependent variables and Act.si/h T and Th as independent variables, with natural killer cells as dependent variables and Act.si/h T and Tsi as independent variables and with OKIa1 antigen positive lymphocytes as dependent variable and monocytes and natural killer cells as independent variables, respectively, whereby the standardized partial regression coefficients (SPRCs) and the multiple correlation coefficients (MCCs) in the respective groups were determined. These results are shown in Table 15 and Figure 6(B).

(2) Secondly, the MRAs (A) - (C) were carried out as in (1) above, using the data of peripheral lymphocyte and monocyte counts obtained in Example 2. These results are shown in Table 16.

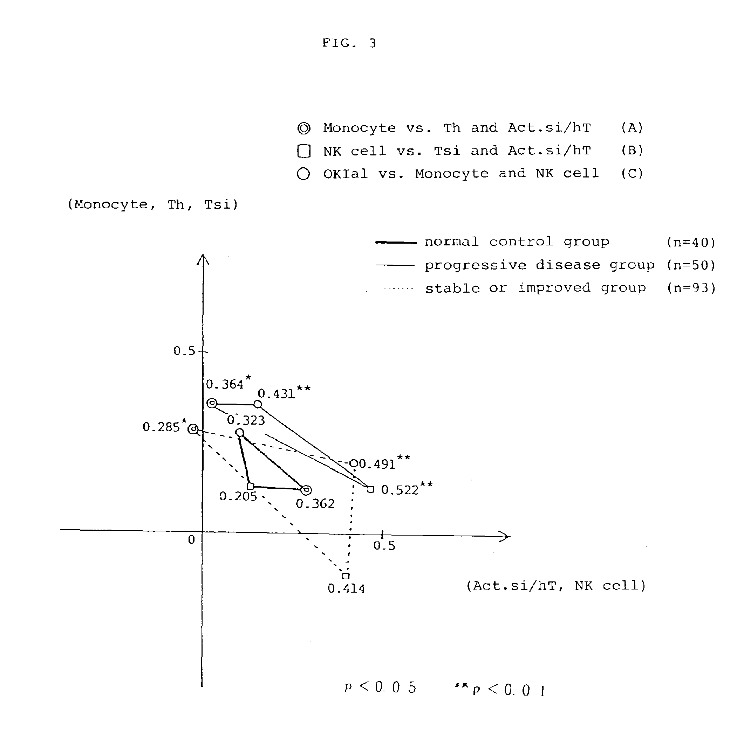

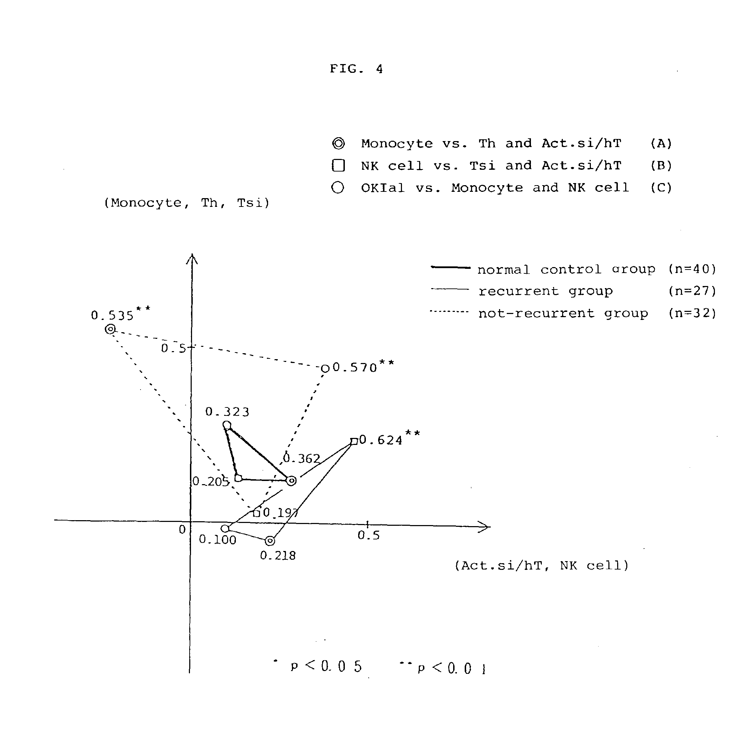

Furthermore, in Figure 3, the numerical values for (A) to (C) shown in Table 15 were separately plotted in respect of the normal control, progressive disease and stable or improved disease groups, respectively. The respective MCCs are shown therein with the numerical values written in. Figure 6(A) is a three-dimensional representation of Figure 3 where the MCCs in Figure 3 are plotted along the hight (Z-axis). Figure 4 is a graph obtained by representing the numerical values in Table 16 as described for Figure 3.

The numerical values shown in Table 15, namely the SPRC of each independent variable and the corresponding MCC obtained by the MRA(C) with OKIa1 antigen positive cells as dependent variables and monocytes and natural killer cells as independent variables, can be converted into the coordinate points on the three-dimensional graph by plotting the respective SPRCs along X and Y axes, respectively, and the MCCs along Z axis. Likewise, the coordinate points of Act.T and Act.B obtained by MRAs(D) and (E) with Act.T and Act.B, respectively, as dependent variables and monocytes and natural killer cells as independent variables, can be plotted for solid representation. These results are shown in Figure 6(B) and the right of Figure 7. In this way, the coordinate points obtained by (C) can be separated into the coordinate points obtained by (D) and (C).

EP 0 443 036 B1

Table 15

|  |  | Normal control group (n=40) | Progressive disease group (n=50) | Stable or improved group (n=93) |
|---|---|---|---|---|
| A | Th* | 0.123497 | 0.357 | 0.288939 |
|  | Act.si/hT* | 0.292302 | 0.025331 | .-0.028511 |
|  | Multiple correlation coefficient | 0.361784 | 0.363762 | 0.284602 |
| B | Tsi* | 0.128355 | 0.130975 | -0.108097 |
|  | Act.si/hT* | 0.135293 | 0.469714 | 0.397981 |
|  | Multiple correlation coefficient | 0.204903 | 0.522209 | 0.413896 |
| C | NK cells* | 0.098971 | 0.150721 | 0.430453 |
|  | Monocytes* | 0.279533 | 0.362853 | 0.197735 |
|  | Multiple correlation coefficient | 0.323077 | 0.430755 | 0.490599 |
|  | NK cells* | 0.122544 | 0.635762 | 0.545705 |
|  | Monocytes* | 0.25562 | -0.151983 | -0.050313 |
|  | Multiple correlation coefficient | 0.314612 | 0.610101 | 0.543195 |
| E | NK cells* | 0.089538 | 0.084333 | 0.330131 |
|  | Monocytes* | 0.263762 | 0.384272 | 0.215153 |
|  | Multiple correlation coefficient | 0.302697 | 0.416229 | 0.410947 |

*   Standardized partial regression coefficient

35

Table 16

| | | Normal control group (n=40) | Recurrent group (n=27) | Not-recurrent group (n=32) |
|---|---|---|---|---|
| A | Th* | 0.123497 | -0.045375 | 0.555845 |
| A | Act.si/hT* | 0.292302 | 0.230123 | -0.235544 |
| A | Multiple correlation coefficient | 0.361784 | 0.21754 | 0.534908 |
| B | Tsi* | 0.128355 | 0.241964 | 0.029923 |
| B | Act.si/hT* | 0.135293 | 0.471792 | 0.192667 |
| B | Multiple correlation coefficient | 0.204903 | 0.623815 | 0.197369 |
| C | NK cells* | 0.098971 | 0.098957 | 0.386759 |
| C | Monocytes* | 0.279533 | -0.014827 | 0.447517 |
| C | Multiple correlation coefficient | 0.323077 | 0.099820 | 0.570277 |

\* Standardized partial regression coefficient

Example 4

For treatments 1-3 in the following case, the standardized partial regression coefficients for immunologic three-factor sets, the partial regression coefficients, the contributions and the multiple correlation coefficients were determined and then the immunokinetics were evaluated.

[A case report of 59 year-old male with bladder cancer]

On June 4, 1987, he was diagnosed as multiple superficial bladder cancer and underwent transurethral resection of bladder tumor (TUR-Bt) and random biopsy. Transitional cell carcinoma with the category of stage pTIb and grade 2 of malignancy was observed, as well as multiple carcinoma in situ in all of ten biopsied specimens.

(Treatment 1)

For the prevention of recurrence, instillation into the urinary bladder of 9 Mega-Unit (MU) of gamma-interferon was initiated one week after the TUR-Bt operation, once a week for the first four weeks and once a month thereafter. But the postoperative urinary cytology became positive 4 months after the operation and he underwent TUR-biopsy of the bladder on November 6, with the result that the recurrence of multiple carcinoma in situ was noted.

On January 29, 1988, a catheter for arterial infusion was settled for the subselective arterial infusion therapy, puncturing the left femoral artery. The five-drug combination chemotherapy (COMPA) consisting of cisplatin, vincristine (trademark: Oncovin), methotrexate, peplomycin and adriamycin was carried out in 3 courses and he obtained a complete response.

(Treatment 2)

From March 15, 1988, after the completion of arterial infusion therapy, the combination of 9MU of gamma-interferon (IFN-$\gamma$) and 200 mcg of muramyl dipeptide(MDP; synthetic product corresponding to the cell wall skeleton of tubercle bacillus) was instilled into the cavity of the urinary bladder. The interval of instillation was the same as that or the single agent instillation of IFN-$\gamma$. From about October, 1988, however, the urinary cytology became positive again and carcinoma in situ recurred.

(Treatment 3)

From December 5, 1988, the combined therapy by subcutaneous injection of 200 mcg of MDP for general sensitization (priming) and instillation into the urinary bladder of 9MU of IFN-$\gamma$, 6MU of IFN-$\alpha$ and 200 mcg of MDP (eliciting) was carried out for the purpose of treatment. The peroral administration of 100 mg/day of cyclophosphamide (trademark: Endoxan) was simultaneously given twice per day (morning and evening) for 2 weeks and repeated every 4 weeks. The priming and the eliciting were carried out once per week (a total of 4 times), twice per two weeks (a total of 8 times) and once per month (a total of nine times). As of the end of August, 1989, the urinary cytology continued to be negative and no evidence to show cancer was noted by cystoscopy, different examinations such as ultrasound and CT-scanning, thus indicating a satisfactory progress.

[The assessment of immunokinetics]

In the same manner as in Example 3, the values for the immunologic three-factor sets were determined in the normal control group and each of treatments 1-3 mentioned above.

The results of lymphocyte subpopulations and monocyte counts for normal controls are shown in Table 17, those of multiple regression analysis of (A) in Table 18, those of multiple regression analysis of (B) in Table 19 and the those of multiple regression analysis of (C) in Table 20.

The results for treatments 1-3 are also shown in the same order as for the normal controls. Thus the results for treatment 1 are shown in Tables 21 to 24, those for treatment 2 in Tables 25 to 28 and those for treatment 3 in Tables 29 to 32.

Table 17

| (n=40) | Unit: /mm$^3$ | |
|---|---|---|
| Th | 423 | ±155 |
| Tsi | 387 | ±170 |
| Act.si/hT | 25.8 | ±19.5 |
| NK Cells | 201 | ±106 |
| Monocytes | 330 | ±142 |
| OKIa1 | 588 | ±310 |

Mean ± SD

Table 18

Dependent variable = Monocytes (n=40)

| Independent variables | Standardized partial regression coefficient | Partial regression coefficient | Contribution (%) |
|---|---|---|---|
| Th | 0.123497 | 0.11272 | 1.26 |
| Act.si/hT | 0.292302 | 2.1213 | 11.83 |
| Constant | | 225.542 | Sum 13.09 |

Multiple correlation coefficient=0.361784 (n.s.)

n.s.: not significant (f-test)

Table 19

Dependent variable = **NK cells** (n=40)

| Independent variables | Standardized partial regression coefficient | Partial regression coefficient | Contribution (%) |
|---|---|---|---|
| Tsi | 0.128355 | 0.0802545 | 1.58 |
| Act.si/hT | 0.135293 | 0.735321 | 2.62 |
| Constant | | 149.825 | Sum   4.20 |

Multiple correlation coefficient=0.204903 (n.s.)

   n.s.:  not significant (f-test)

Table 20

Dependent variable = OKIal   (n=40)

| Independent variables | Standardized partial regression coefficient | Partial regression coefficient | Contribution (%) |
|---|---|---|---|
| **NK cells** | 0.0989708 | 0.289003 | 0.89 |
| Monocytes | 0.279533 | 0.611309 | 9.54 |
| Constant | | 328.051 | Sum   10.43 |

Multiple correlation coefficient=0.323077 (n.s.)

   n.s.:  not significant (f-test)

39

Table 21

| (n=7) | Unit: /mm$^3$ | |
|---|---|---|
| Th | 574 | ±119 |
| Tsi | 651 | ±253 |
| Act.si/hT | 45.9 | ±22.2 |
| **NK cells** | 237 | ±177 |
| Monocytes | 514 | ±334 |
| OKIal | 807 | ±322 |

Mean ± SD

Table 22

Dependent variable = Monocytes (n=7)

| Independent variables | Standardized partial regression coefficient | Partial regression coefficient | Contribution (%) |
|---|---|---|---|
| Th | 0.133871 | 0.376401 | 1.74 |
| Act.si/hT | −0.206615 | −3.11349 | 3.39 |
| Constant | | 440.99 | Sum 5.13 |

Multiple correlation coefficient=0.226519 (n.s.)

n.s.: not significant (f-test)

Table 23

Dependent variable = **NK cells** (n=7)

| Independent variables | Standardized partial regression coefficient | Partial regression coefficient | Contribution (%) |
|---|---|---|---|
| Tsi | 0.32915 | 0.229814 | 10.29 |
| Act.si/hT | 0.737381 | 5.88337 | 44.05 |
| Constant | | -182.243 | Sum   54.34 |

Multiple correlation coefficient=0.737155 (n.s.)

n.s.:   not significant (f-test)

Table 24

Dependent variable = OKIal   (n=7)

| Independent variables | Standardized partial regression coefficient | Partial regression coefficient | Contribution (%) |
|---|---|---|---|
| **NK cells** | -0.394324 | -0.718647 | 15.03 |
| Monocytes | -0.629799 | -0.607734 | 31.09 |
| Constant | | 1290.32 | Sum   46.12 |

Multiple correlation coefficient=0.679087 (n.s.)

n.s.:   not significant (f-test)

Table 25

| (n=5) | Unit: /mm$^3$ | |
|---|---|---|
| Th | 468 | ±108 |
| Tsi | 374 | ±172 |
| Act.si/hT | 48.9 | ±18.3 |
| **NK cells** | 294 | ±122 |
| Monocytes | 580 | ±259 |
| OKIal | 289 | ± 68 |

Mean ± SD

Table 26

Dependent variable = Monocytes (n=5)

| Independent variables | Standardized partial regression coefficient | Partial regression coefficient | Contribution (%) |
|---|---|---|---|
| Th | -0.937248 | -0.937248 | 14.11 |
| Act.si/hT | 0.0423683 | 0.598427 | 0.15 |
| Constant | | 989.381 | Sum 14.26 |

Multiple correlation coefficient=0.37768 (n.s.)

n.s.: not significant (f-test)

Table 27

Dependent variable = **NK cells** (n=5)

| Independent variables | Standardized partial regression coefficient | Partial regression coefficient | Contribution (%) |
|---|---|---|---|
| Tsi | 1.15961 | 0.817615 | 59.23 |
| Act.si/hT | -1.43699 | -9.53279 * | 32.44 |
| Constant | | 453.975 | Sum 91.67 |

Multiple correlation coefficient=0.957419 (n.s.)

  * : p<0.05 (t-test)     n.s.: not significant (f-test)

Table 28

Dependent variable = OKIal   (n=5)

| Independent variables | Standardized partial regression coefficient | Partial regression coefficient | Contribution (%) |
|---|---|---|---|
| **NK cells** | 0.831561 | 0.462259 | 32.55 |
| Monocytes | 0.425539 | 0.111103 | 11.29 |
| Constant | | 88.5486 | Sum 43.84 |

Multiple correlation coefficient=0.662119 (n.s.)

  n.s.: not significant (f-test)

Table 29

| (n=8) | Unit: /mm$^3$ | |
|---|---|---|
| Th | 371 | ± 76 |
| Tsi | 405 | ± 77 |
| Act.si/hT | 42.4 | ±32.5 |
| **NK cell**s | 285 | ±112 |
| Monocytes | 405 | ±198 |
| OKIa1 | 477 | ±181 |

Mean ± SD

Table 30

Dependent variable = Monocytes (n=8)

| Independent variables | Standardized partial regression coefficient | Partial regression coefficient | Contribution (%) | |
|---|---|---|---|---|
| Th | 0.0003 | 0.000784 | | 0.00 |
| Act.si/hT | 0.285619 | 1.73822 | | 8.16 |
| Constant | | 330.677 | Sum | 8.16 |

Multiple correlation coefficient=0.285681 (n.s.)

n.s.: not significant (f-test)

Table 31

Dependent variable = NK cells (n=8)

| Independent variables | Standardized partial regression coefficient | Partial regression coefficient | Contribution (%) |
|---|---|---|---|
| Tsi | 0.178866 | 0.261182 | 2.73 |
| Act.si/hT | 0.741462 | 2.55246 | 45.31 |
| Constant | | 71.4357 | Sum 48.04 |

Multiple correlation coefficient=0.693139 (n.s.)

n.s.: not significant (f-test)

Table 32

Dependent variable = OKIa1 (n=8)

| Independent variables | Standardized partial regression coefficient | Partial regression coefficient | Contribution (%) |
|---|---|---|---|
| NK cells | 0.416353 | 0.676139 | 22.17 |
| Monocytes | 0.262873 | 0.241476 | 6.61 |
| Constant | | 186.84 | Sum 28.78 |

Multiple correlation coefficient=0.536508 (n.s.)

n.s.: not significant (f-test)

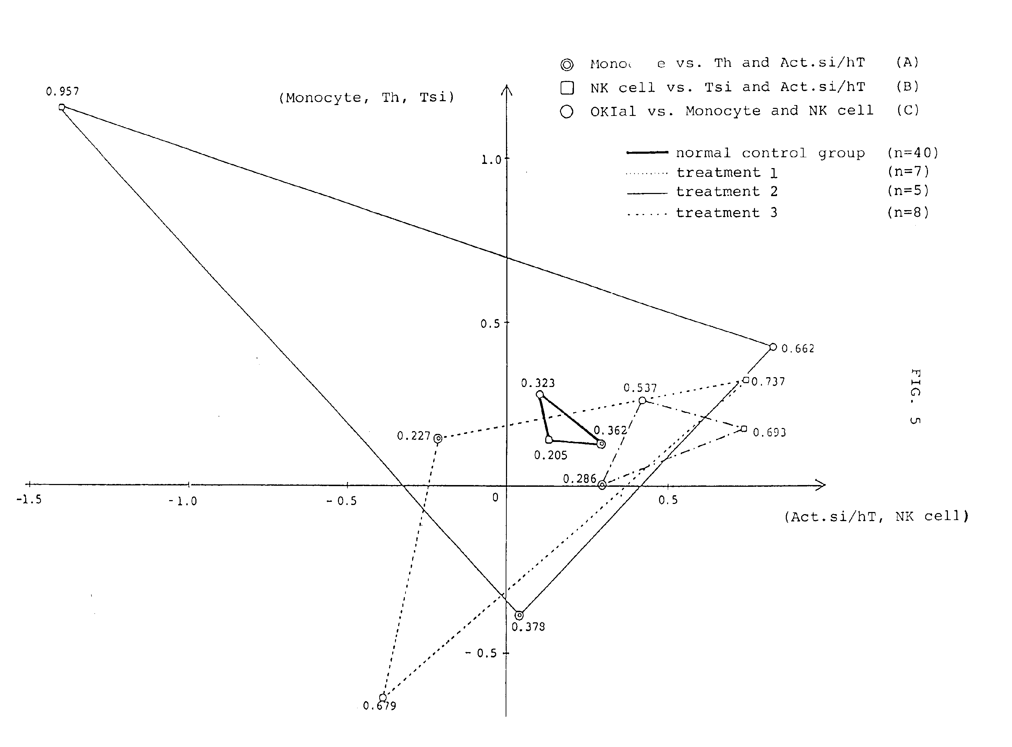

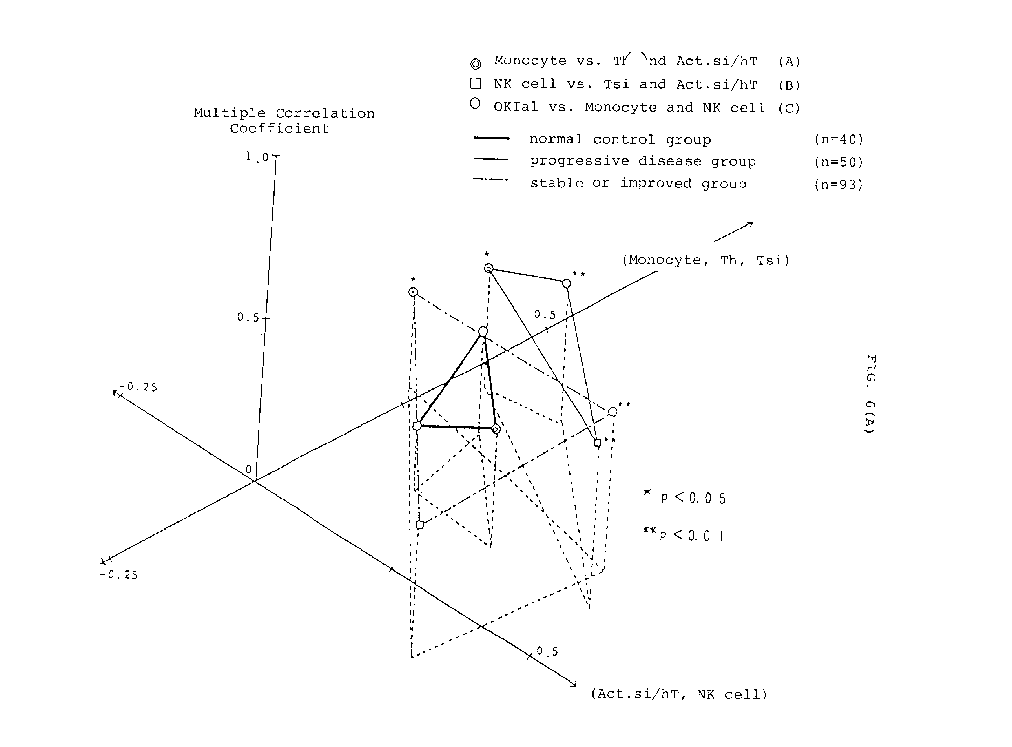

Furthermore, in Figure 5 is shown a graph indicating the change of correlated functions of the three-factor sets obtained with the normal control group and treatments 1 - 3.

It has been confirmed from the results mentioned above that the approaching of the dispersion, at the initiation of treatment, of the immunologic three-factor sets to the normal control's pattern in the course of immunotherapy is indicative of healing.

Example 5

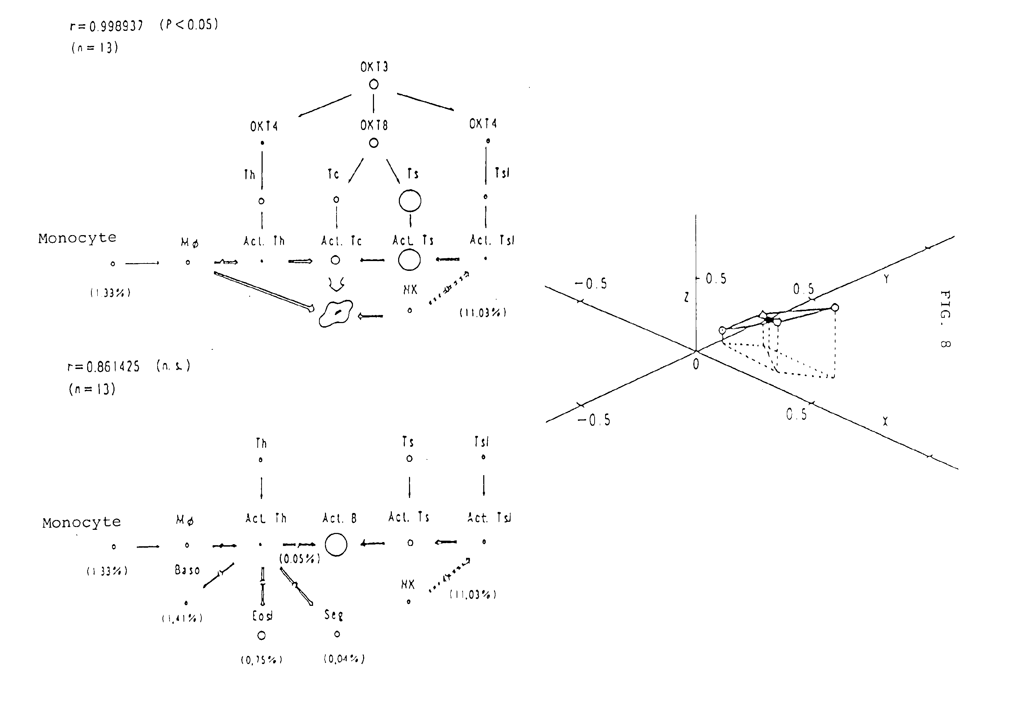

The results of a total of thirteen measurements carried out every 2 or 3 days in a normal healthy 49 year-old male and the cellular immunokinematograms obtained therefrom are shown in Tables 33 to 42 and Figure 8, respectively.

In the upper-left of Figure 8 shows the T cell immunokinematogram. The symbols in Figure 8 is shown used to mean the following: →:process of differentiation or maturation; ⟹ : non-functioning or impaired antigen recognition mechanism; ⟹ : no suppressive function of natural killer cells; ⟹ : accelerating or promoting function; ⟶ : suppression; ⟹ and ⟹ : cytotoxic function; ● : 0.1% ≧ of contribution. The values in % described in the lower part of this upper-left figure are for the contribution of activated suppression inducer/helper T cells versus monocytes or natural killer cells.

The B cell immunokinematogram is shown in the lower-left of Figure 8. The symbols in the figure are used to mean the following: ⟹ neither accelerating nor promoting; ⟶ : suppression; Baso: basophils; Eosi: eosinophils; Seg: neutrophils.

The immunologic three-factor sets are shown in the right of Figure 8. The symbols in the figure are used to mean the following: Δ : the correlation of monocyte versus helper and activated suppression inducer/helper T cells; □ : the correlation of natural killer cells versus suppression inducer and activated suppression inducer/helper T cells; ● : the correlation of OKIa1 antigen positive (activated) lymphocytes versus monocytes and natural killer cells, ○: the correlation of activated T lymphocytes versus monocytes and natural killer cells; ◎ : the correlation of activated B lymphocytes versus monocytes and natural killer cells. This example is to demonstrate the possibility of drawing specific immunokinematograms peculiar to individuals.

It has now been confirmed with this example that specific immunokinematograms peculiar to individuals can be drawn.

Example 6

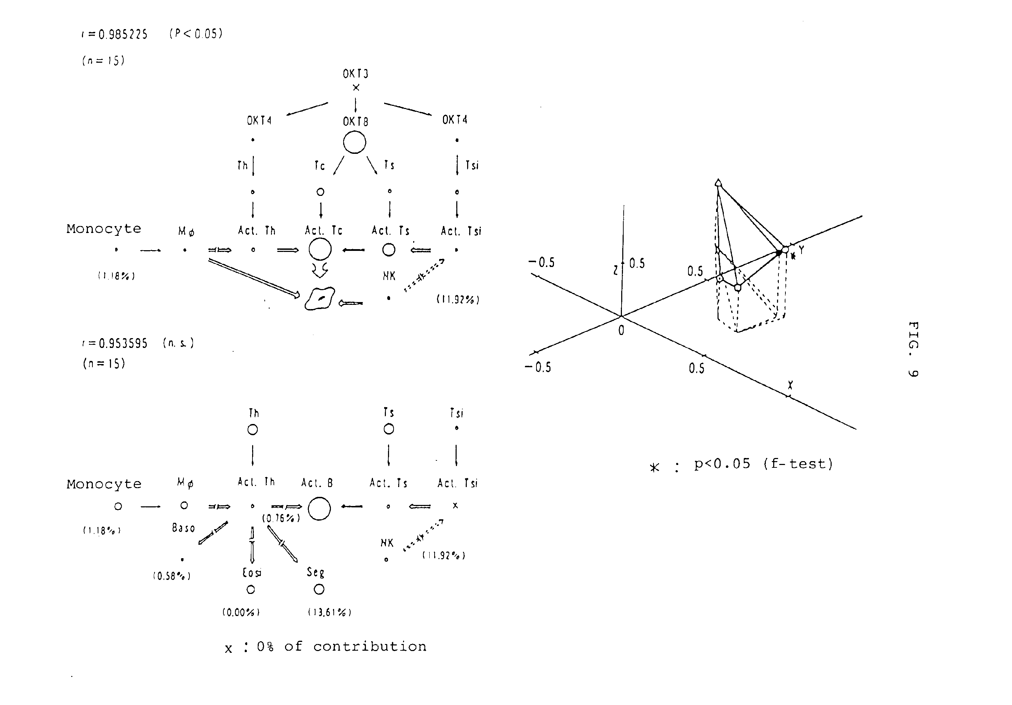

Measurements were carried out on three patients with chronic rheumatoid arthritis to draw T and B cell immunokinematograms and a graph of immunologic three-factor sets. The results are shown in Tables 43 to 52 and Figure 9. One of the cases, a 59 year-old male, is under treatment and has a stable and improved clinical course 4 months later after the onset of disease. The other cases, 77 and 83 year-old females, had undergone treatment for 8 and 10-odd years, respectively, but are no longer under treatment because of the state of disease being stable and improved. The multiple regression analysis was carried out in the respective cases on the data from 3 x 5 (15 in total) measurements carried out once every week or 2 weeks.

From these results, it was confirmed that the stable or improved state of disease was due to the immune state being well controlled up to a normal healthy level.

Tables 33-52 shown below are as follows:

Table 33. The results of lymphocyte subpopulations, monocytes and granulocytes (basophils, eosinophils and neutrophils) counts in the peripheral blood of the 49 y.o. healthy male (n=13)

Table 34. The results of multiple regression analysis of the data in Table 33, using activated suppressor/cytotoxic T cells as dependent variable

Table 35. The multiple regression analysis between monocytes helper T cells and activated suppression inducer/helper T cells

Table 36. The multiple regression analysis between natural killer cells, suppression inducer T cells and activated suppression inducer/helper T cells

Table 37. The standardized partial regression coefficients and multiple correlation coefficients for preparing a graph of immunologic three-factor sets

Table 38. The results of multiple regression analysis using activated B cells as dependent variable

Table 39. The multiple regression analysis between activated B cells, helper T cells and activated suppression inducer/helper T cells

Table 40. The multiple regression analysis between basophils, helper T cells and activated suppression inducer/helper T cells

Table 41. The multiple regression analysis between eosinophils, helper T cells and activated suppression inducer/helper T cells

Table 42. The multiple regression analysis between neutrophils helper T cells and activated suppression inducer/helper T cells

Table 43. The results of lymphocyte subpopulations, monocyte and granulocytes counts in the peripheral blood

46

of the 59 y.o. male with chronic rheumatoid arthritis under treatment, and of the 77 y.o. and 83 y.o. females with it under no treatment, all these three cases having good clinical courses during the measurements Table 44. The results of multiple regression analysis using activated suppressor/cytotoxic T cells as dependent variable

Table 45. The multiple regression analysis between monocytes helper T cells and activated suppression inducer/helper T cells

Table 46. The multiple regression analysis between natural killer cells, suppression inducer T cells and activated suppression inducer/helper T cells

Table 47. The standardized partial regression coefficients and multiple correlation coefficients for preparing a graph of immunologic three-factor sets

Table 48. The results of multiple regression analysis using activated B cells as dependent variable

Table 49. The multiple regression analysis between activated B cells, helper T cells and activated suppression inducer/helper T cells

Table 50. The multiple regression analysis between basophils helper T cells and activated suppression inducer/helper T cells

Table 51. The multiple regression analysis between eosinophils, helper T cells and activated suppression inducer/helper T cells

Table 52. The multiple regression analysis between neutrophils, helper T cells and activated suppression inducer/helper T cells

Table 33

Unit : / mm$^3$          (n=13)

| | | | |
|---|---|---|---|
| OKT 3 | 1867 | ± | 281 |
| OKT 4 | 1084 | ± | 174 |
| OKT 8 | 689 | ± | 157 |
| OKIal | 614 | ± | 184 |
| Th | 263 | ± | 84 |
| Tsi | 786 | ± | 133 |
| Tc | 458 | ± | 82 |
| Ts | 279 | ± | 117 |
| Act.si/hT | 89.0 | ± | 24.5 |
| Act.s/c T | 143.5 | ± | 61.4 |
| **NK cells** | 49 | ± | 17 |
| Monocytes | 130 | ± | 53 |
| Act.T | 232.5 | ± | 69.2 |
| Act.B | 381 | ± | 147 |
| Basophils | 25 | ± | 21 |
| Eosinophils | 266 | ± | 86 |
| Neutrophils | 2489 | ± | 517 |

Mean ± SD

Table 34

| Independent variables | Dependent variable — Act.s/c T | |
| --- | --- | --- |
| | Partial regression coefficient | Contribution (%) |
| OKT3 | −0.370896* | 5.64 |
| OKT4 | 0.238379 | 0.16 |
| OKT8 | −0.508682 | 12.67 |
| Th | 0.308995 | 4.42 |
| Tsi | −0.025641 | 1.91 |
| Tc | 0.60887 | 4.48 |
| Ts | 1.36588** | 69.07 |
| Act.si/hT | 0.187919 | 0.04 |
| NK cells | 0.376798 | 0.16 |
| Monocytes | −0.216849 | 1.25 |
| Constant | 199.631 | (Sum) 99.80 |
| Multiple correlation coefficient | 0.998937 (p < 0.05) | |

*p < 0.05, **p < 0.01 (t-test)

Table 35

| Dependent variable | Monocytes | |
|---|---|---|
| Independent variables | Partial regression coefficient | Contribution (%) |
| Th | 0.107463 | 2.97 |
| Act.si/hT | 0.252071 | 1.33 |
| | | (Sum) |
| Constant | 79.2156 | 4.30 |
| Multiple correlation coefficient | 0.207305 (n.s.) | |

n.s.: not significant (f-test)

Table 36

| Dependent variable | NK cells | |
|---|---|---|
| Independent variables | Partial regression coefficient | Contribution (%) |
| Tsi | 0.009091 | 0.20 |
| Act.si/hT | 0.190682 | 11.03 |
| | | (Sum) |
| Constant | 24.4186 | 11.23 |
| Multiple correlation coefficient | 0.33518 (n.s.) | |

n.s.: not significant (f-test)

Table 37

| | | Standardized partial regression coefficient |
|---|---|---|
| A | Th | 0.168394 |
| | Act.si/ht | 0.115387 |
| | Multiple correlation coefficient | 0.207305 |
| B | Tsi | 0.071702 |
| | Act.si/hT | 0.276463 |
| | Multiple correlation coefficient | 0.33518 |
| C | NK cells | 0.181317 |
| | Monocytes | 0.132155 |
| | Multiple correlation coefficient | 0.234756 |
| D | NK cells | 0.435111 |
| | Monocytes | 0.162986 |
| | Multiple correlation coefficient | 0.479583 |
| E | NK cells | 0.021822 |
| | Monocytes | 0.088407 |
| | Multiple correlation coefficient | 0.093145 |

Table 38

| Dependent variable / Independent variables | Act.B | |
|---|---|---|
| | Partial regression coefficient | Contribution (%) |
| Th | -0.227762 | 0.11 |
| Tsi | -0.286855 | 1.55 |
| Tc | 2.18391 | 8.95 |
| Ts | -1.59677 | 8.42 |
| Act.si/hT | 0.869905 | 0.20 |
| Act.s/c T | 2.75868 | 15.66 |
| NK cells | 4.82383 | 2.50 |
| Monocytes | 1.29852 | 5.18 |
| Neutrophils | 0.056596 | 12.51 |
| Basophils | -3.97208 | 3.27 |
| Eosinophils | -0.510105 | 15.85 |
| Constant | -668.549 | (Sum) 74.20 |
| Multiple correlation coefficient | 0.861425(n.s.) | |

n.s.: not significant (f-test)

Table 39

| Dependent variable | Act.B | |
|---|---|---|
| Independent variables | Partial regression coefficient | Contribution (%) |
| Th | 0.11349 | 0.43 |
| Act.si/hT | 0.139013 | 0.05 |
| | | (Sum) |
| Constant | 339.132 | 0.48 |
| Multiple correlation coefficient | 0.069296(n.s.) | |

n.s.: not significant (f-test)

Table 40

| Dependent variable | Basophils | |
|---|---|---|
| Independent variables | Partial regression coefficient | Contribution (%) |
| Th | 0.067288 | 7.41 |
| Act.si/hT | 0.102045 | 1.41 |
| | | (Sum) |
| Constant | -1.3228 | 8.82 |
| Multiple correlation coefficient | 0.29705(n.s.) | |

n.s.: not significant (f-test)

Table 41

| | Dependent variable | Eosinophils | |
|---|---|---|---|
| Independent variables | | Partial regression coefficient | Contribution (%) |
| Th | | -0.040742 | 0.16 |
| Act.si/hT | | -0.299355 | 0.75 |
| | | | (Sum) |
| Constant | | 303.671 | 0.91 |
| Multiple correlation coefficient | | 0.095016(n.s.) | |

n.s.: not significant (f-test)


Table 42

| | Dependent variable | Neutrophils | |
|---|---|---|---|
| Independent variables | | Partial regression coefficient | Contribution (%) |
| Th | | -1.21932 | 3.93 |
| Act.si/hT | | -0.408117 | 0.04 |
| | | | (Sum) |
| Constant | | 2846.18 | 3.97 |
| Multiple correlation coefficient | | 0.199176(n.s.) | |

n.s.: not significant (f-test)

Table 43

Unit: /mm$^3$

| | 59 y.o. male (n=5) | | 77 y.o. female (n=5) | | 83 y.o. female (n=5) | | Sum (n=15) | |
|---|---|---|---|---|---|---|---|---|
| OKT3 | 1412 | ± 191 | 1008 | ± 214 | 1356 | ± 491 | 1259 | ± 356 |
| OKT4 | 1008 | ± 133 | 642 | ± 144 | 1246 | ± 448 | 965 | ± 367 |
| OKT8 | 544 | ± 136 | 440 | ± 126 | 196 | ± 62 | 393 | ± 184 |
| OKIal | 510 | ± 361 | 534 | ± 140 | 258 | ± 112 | 434 | ± 251 |
| Th | 590 | ± 29 | 356 | ± 139 | 162 | ± 82 | 369 | ± 201 |
| Tsi | 372 | ± 101 | 252 | ± 99 | 988 | ± 307 | 537 | ± 380 |
| Tc | 280 | ± 84 | 292 | ± 101 | 86 | ± 26 | 219 | ± 121 |
| Ts | 292 | ± 94 | 154 | ± 61 | 120 | ± 49 | 189 | ± 101 |
| Act.si/hT | 166.8 ± | 38.4 | 79.8 ± | 15.2 | 54.3 ± | 27.5 | 100.3 | ± 56.5 |
| Act.s/c T | 246.7 ± | 50.4 | 242.3 ± | 98.6 | 61.2 ± | 20.4 | 183.4 | ± 107.8 |
| NK cells | 38 | ± 14 | 43 | ± 16 | 35 | ± 22 | 39 | ± 17 |
| Monocytes | 339 | ± 196 | 179 | ± 106 | 120 | ± 66 | 213 | ± 157 |
| Act.T | 413.5 ± | 68.8 | 322.1 ± 111.6 | | 115.5 ± | 44.0 | 283.7 | ± 148.7 |
| Act.B | 138 | ± 269 | 214 | ± 160 | 143 | ± 76 | 165 | ± 176 |
| Basophils | 11 | ± 11 | 19 | ± 25 | 14 | ± 23 | 15 | ± 20 |
| Eosinophils | 66 | ± 58 | 114 | ± 28 | 53 | ± 13 | 78 | ± 44 |
| Neutrophils | 4123 | ± 509 | 3093 | ± 448 | 5148 | ± 1096 | 4121 | ± 1109 |

Mean ± SD

Table 44

| Independent variables | Dependent variable | Act.s/c T | |
|---|---|---|---|
| | | Partial regression coefficient | Contribution (%) |
| OKT3 | | 0.013271 | 0.00 |
| OKT4 | | 0.393968 | 0.63 |
| OKT8 | | -0.013259 | 78.52 |
| Th | | -0.027523 | 1.11 |
| Tsi | | -0.581591 | 2.58 |
| Tc | | 0.037913 | 6.87 |
| Ts | | 0.643833 | 2.14 |
| Act.si/hT | | -1.50839* | 3.87 |
| NK cells | | 0.960777 | 1.10 |
| Monocytes | | -0.069336 | 0.24 |
| Constant | | 113.464 | (Sum) 97.06 |
| Multiple correlation coefficient | | 0.985225 (p < 0.05) | |

*p < 0.05 (t-test)

Table 45

| Dependent variable | Monocytes | |
|---|---|---|
| Independent variables | Partial regression coefficient | Contribution (%) |
| Th | 0.590448 | 32.73 |
| Act.si/hT | -0.5998 | 1.18 |
| | | (Sum) |
| Constant | 54.829 | 33.91 |
| Multiple correlation coefficient | 0.582243 (n.s.) | |

n.s.: not significant (f-test)

Table 46

| Dependent variable | NK cells | |
|---|---|---|
| Independent variables | Partial regression coefficient | Contribution (%) |
| Tsi | 0.011200 | 5.50 |
| Act.si/hT | 0.130613 | 11.92 |
| | | (Sum) |
| Constant | 19.4799 | 17.42 |
| Multiple correlation coefficient | 0.417328 (n.s.) | |

n.s.: not significant (f-test)

Table 47

|  |  | Standardized partial regression coefficient |
|---|---|---|
| A | Th | 0.759351 |
| | Act.si/ht | -0.216429 |
| | Multiple correlation coefficient | 0.582243 |
| B | Tsi | 0.251576 |
| | Act.si/hT | 0.43663 |
| | Multiple correlation coefficient | 0.417328 |
| C | NK cells | 0.445037 |
| | Monocytes | 0.478064 |
| | Multiple correlation coefficient | 0.602544 |
| D | NK cells | 0.461599 |
| | Monocytes | 0.507469 |
| | Multiple correlation coefficient | 0.632957* |
| E | NK cells | 0.289039 |
| | Monocytes | 0.282588 |
| | Multiple correlation coefficient | 0.372834 |

$*p < 0.05$ (f-test)

Table 48

| Independent variables | Dependent variable — Act.B | |
| --- | --- | --- |
| | Partial regression coefficient | Contribution (%) |
| Th | -1.2468 | 11.21 |
| Tsi | 0.060024 | 0.13 |
| Tc | 0.056351 | 0.01 |
| Ts | 1.30477 | 20.60 |
| Act.si/hT | 0.183446 | 0.06 |
| Act.s/c T | 0.784022 | 3.00 |
| NK cells | 0.937972 | 5.30 |
| Monocytes | 0.956598 | 14.43 |
| Neutrophils | 0.081444 | 23.64 |
| Basophils | -1.16985 | 0.74 |
| Eosinophils | 4.65044 | 11.82 |
| Constant | -747.59 | (Sum) 90.94 |
| Multiple correlation coefficient | 0.953595 (n.s.) | |

n.s.:  not significant (f-test)

Table 49

| Independent variables \ Dependent variable | Act.B | |
|---|---|---|
| | Partial regression coefficient | Contribution (%) |
| Th | -0.327244 | 3.52 |
| Act.si/hT | 1.28033 | 0.76 |
| | | (Sum) |
| Constant | 157.501 | 4.28 |
| Multiple correlation coefficient | 0.206793(n.s.) | |

n.s.:  not significant (f test)

Table 50

| Independent variables \ Dependent variable | Basophils | |
|---|---|---|
| | Partial regression coefficient | Contribution (%) |
| Th | -0.031931 | 3.81 |
| Act.si/hT | 0.052967 | 0.58 |
| | | (Sum) |
| Constant | 21.2798 | 4.39 |
| Multiple correlation coefficient | 0.209473(n.s.) | |

n.s.:  not significant (f-test)

Table 51

| Independent variables \ Dependent variable | Eosinophils | |
|---|---|---|
| | Partial regression coefficient | Contribution (%) |
| Th | -0.002991 | 0.01 |
| Act.si/hT | 0.013110 | 0.00 |
| | | (Sum) |
| Constant | 77.6568 | 0.01 |
| Multiple correlation coefficient | 0.008404 (n.s.) | |

n.s.:  not significant (f-test)

Table 52

| Independent variables \ Dependent variable | Neutrophils | |
|---|---|---|
| | Partial regression coefficient | Contribution (%) |
| Th | -5.00489 | 7.38 |
| Act.si/hT | 14.4518 | 13.61 |
| | | (Sum) |
| Constant | 4520.23 | 20.99 |
| Multiple correlation coefficient | 0.458165 (n.s.) | |

n.s.:  not significant (f-test)

[Brief Description of Drawings]

Figure 1.        The cellular immunokinematograms obtained in Example 1
Figure 2.        The cellular immunokinematograms obtained in Example 2
Figure 3.        The correlated graphs of immunologic three-factor sets obtained from Example 1 in accordance with Example 3
Figure 4.        The correlated graphs of immunologic three-factor sets obtained from Example 2 in accordance with Example 3
Figure 5.        The correlated graphs of immunologic three-factor sets obtained in accordance with Example 4
Figure 6(A).     The three-dimmensional representations of Figure 3
Figure 6(B).     The three-dimmensional graphs of the correlations of T and B cell immunologic three-factor sets obtained in Example 3(1)

61

Figure 7.        T cell immunokinematogram, immunologic three-factor set and T and B cell immunologic three-factor sets on the 40 normal control group of Examples 1 and 2

Figure 8.        T cell immunokinematogram, immunologic three-factor set and T and B cell immunologic three-factor sets on the 49 y.o. healthy male of Example 5

Figure 9.        T and B cell immunokinematograms, immunologic three-factor set and T and B cell immunologic three-factor sets on the three chronic rheumatoid arthritis patients of Example 6

## Claims

1. A method of measuring immunokinetics through blood analysis, characterized by carrying out the following process steps (a) - (f) to assess the antigen recognition mechanism:

   a) The counts of monocytes, helper T lymphocytes and the subset of activated suppression inducer/helper T lymphocytes in blood samples are measured;

   b) The multiple regression analysis using monocytes as dependent variables and helper and the subset of activated suppression inducer/helper T lymphocytes as independent variables is carried out;

   c) The respective partial regression coefficients of helper and the subset of activated suppression inducer/helper T lymphocytes are determined and the significances are tested;

   d) The multiple correlation coefficient is determined and the significance is tested;

   e) The contributions of helper and the subset of activated suppression inducer/helper T lymphocytes are determined; and

   f) The correlations between monocytes, helper T lymphocytes and the subset of activated suppression inducer/helper T lymphocytes are determined.

2. A method of measuring immunokinetics through blood analysis, characterized by carrying out the following process steps (a) - (f) to assess the suppressive function of natural killer cells on suppression inducer T lymphocytes:

   a) The counts of natural killer cells, suppression inducer T lymphocytes and the subset of activated suppression inducer/helper T lymphocytes in blood samples are measured;

   b) The multiple regression analysis using natural killer cells as dependent variables and suppression inducer and the subset of activated suppression inducer/helper T lymphocytes as independent variables is carried out;

   c) The respective partial regression coefficients of suppression inducer and the subset of activated suppression inducer/helper T lymphocytes are determined and the significances are tested;

   d) The multiple correlation coefficient is determined and the significance is tested;

   e) The contributions of suppression inducer and the subset of activated suppression inducer/helper T lymphocytes are determined; and

   f) The correlations between natural killer cells, suppression inducer and the subset of activated suppression inducer/helper T lymphocytes are determined.

3. A method of measuring immunokinetics through blood analysis, characterized by carrying out the following process steps (a) - (f) to assess the function of natural killer cells and monocytes against OKIa1 antigen positive lymphocytes;

   a) The counts of OKIa1 antigen positive lymphocytes, natural killer cells and monocytes in blood samples are measured;

   b) The multiple regression analysis using OKIa1 antigen positive lymphocytes as dependent variables and using natural killer cells and monocytes as independent variables is carried out;

   c) The respective partial regression coefficients of natural killer cells and monocytes are determined and the significances are tested;

   d) The multiple correlation coefficient is determined and the significance is tested;

   e) The contributions of natural killer cells and monocytes are determined; and

   f) The correlations between OKIa1 antigen positive lymphocytes, natural killer cells and monocytes are determined.

4. A method of measuring immunokinetics through blood analysis, characterized by carrying out the following process steps (a) - (f) to assess the function of natural killer cells and monocytes on activated suppression inducer/helper and activated suppressor/cytotoxic T lymphocytes:

   a) The counts of activated suppression inducer/helper and activated suppressor/cytotoxic T lymphocytes, natural killer cells and monocytes in blood samples are measured:

b) The multiple regression analysis using the sum of activated suppression inducer/helper and activated suppressor/cytotoxic T lymphocytes as dependent variables and natural killer cells and monocytes as independent variables is carried out;

c) The respective partial regression coefficients of natural killer cells and monocytes are determined and the significances are tested;

d) The multiple correlation coefficient is determined and the significance is tested;

e) The contributions of natural killer cells and monocytes are determined; and

f) The correlations of the sum of activated suppression inducer/helper and activated suppressor/cytotoxic T lymphocytes with natural killer cells and monocytes are determined.

5. A method of measuring immunokinetics through blood analysis, characterized by carrying out the following process steps (a) - (g) to assess the function of natural killer cells and monocytes on activated B lymphocytes;

a) The counts of monocytes, natural killer cells, OKIa1 antigen positive lymphocytes, and the subset of activated suppression inducer/helper and the subset of activated suppressor/cytotoxic T lymphocytes in blood samples are measured;

b) Activated B lymphocytes count is calculated by subtracting from the count of OKIa1 antigen positive lymphocytes that of the subset of activated suppression inducer/helper T lymphocytes and that of the subset of activated suppressor/cytotoxic T lymphocytes;

c) The multiple regression analysis using activated B lymphocytes as dependent variables and natural killer cells and monocytes as independent variables is carried out;

d) The respective partial regression coefficients of natural killer cells and monocytes are determined and the significances are tested;

e) The multiple correlation coefficient is determined and the significance is tested;

f) The contributions of natural killer cells and monocytes are determined; and

g) The correlations of activated B lymphocytes with natural killer cells and monocytes are determined.

6. A method of measuring immunokinetics through blood analysis, characterized by carrying out the following process steps (a) - (d) to obtain standardized partial regression coefficients and multiple correlation coefficients and plotting the coefficients on coordinates to prepare graphs for assessment :

a) The counts of monocytes, natural killer cells, OKIa1 antigen positive lymphocytes, helper and suppression inducer T lymphocytes and the subset of activated suppression inducer/helper T lymphocytes in blood samples are measured;

b) The standardized partial regression coefficients of helper and the subset of activated suppression inducer/helper T lymphocytes are determined by the multiple regression analysis, using monocytes as dependent variables and helper and the subset of activated suppression inducer/helper T lymphocytes as independent variables, to obtain the correlations of monocytes with helper and the subset of activated suppression inducer/helper T lymphocytes;

c) The standardized partial regression coefficients of suppression inducer and the subset of activated suppression inducer/helper T lymphocytes are determined by the multiple regression analysis using natural killer cells as dependent variables and suppression inducer and the subset of activated suppression inducer/helper T lymphocytes as independent variables to obtain the correlations of natural killer cells with suppression inducer and the subset of activated suppression inducer/helper T lymphocytes; and

d) The standardized partial regression coefficients of natural killer cells and monocytes are determined by the multiple regression analysis using OKIa1 antigen positive lymphocytes as dependent variables and natural killer cells and monocytes as independent variables to obtain the correlations of OKIa1 antigen positive lymphocytes with natural killer cells and monocytes.

7. A method of measuring immunokinetics through blood analysis, characterized carrying out the following process steps (a) - (f) to obtain standardized partial regression coefficients and multiple correlation coefficients and plotting the coefficients on coordinates to prepare graphs for assessment:

a) The counts of monocytes, natural killer cells, OKIa1 antigen positive lymphocytes, the subset of activated suppression inducer/helper and the subset of activated suppressor/cytotoxic T lymphocytes and helper and suppression inducer T lymphocytes in blood samples are measured;

b) The standardized partial regression coefficients of helper and the subset of activated suppression inducer/helper T lymphocytes are determined by the multiple regression analysis using monocytes as dependent variables and helper and the subset of activated suppression inducer/helper T lymphocytes as independent variables to obtain the correlations of monocytes with helper and the subset of activated suppression inducer/helper T lymphocytes;

c) The standardized partial regression coefficients of suppression inducer and the subset of activated suppression inducer/helper T lymphocytes are determined by the multiple regression analysis using natural killer cells as dependent variables and suppression inducer and the subset of activated independent inducer/helper T lymphocytes as independent variables to obtain the correlations of natural killer cells with suppression inducer and the subset of activated suppression inducer/helper T lymphocytes;

d) The standardized partial regression coefficients of natural killer cells and monocytes are determined by the multiple regression analysis using OKIa1 antigen positive lymphocytes as dependent variables and natural killer cells and monocytes as independent variables to obtain the correlations of OKIa1 antigen positive lymphocytes with natural killer cells and monocytes;

e) The standardized partial regression coefficients of natural killer cells and monocytes are determined by the multiple regression analysis using the sum of the subset of activated suppression inducer/helper and the subset of activated suppressor/cytotoxic T lymphocytes as dependent variables and natural killer cells and monocytes as independent variables to obtain the correlations of activated T lymphocytes with natural killer cells and monocytes; and

f) Activated B lymphocytes count is calculated by subtracting the counts of the subset of activated suppression inducer/helper and the subset of activated suppressor/cytotoxic T lymphocytes from the OKIa1 antigen positive lymphocyte count and the standardized partial regression coefficients of natural killer cells and monocytes are determined by the multiple regression analysis using activated B lymphocytes as independent variables and natural killer cells and monocytes as independent variables to obtain the correlations of activated B lymphocytes with natural killer cells and monocytes.

8. A method of measuring and assessing immunokinetics through blood analysis, characterized by carrying out the following process steps (a) - (f) and assessing integrated T cell immunokinetics on the basis of the resultant T cell immunokinematograms:

a) The counts of CD3, CD4 and CD8 antigen positive lymphocytes, helper, suppression inducer, suppressor and cytotoxic T lymphocytes, the subset of activated suppression inducer/helper and the subset of activated suppressor/cytotoxic T lymphocytes, natural killer cells and monocytes in blood samples are measured;

b) The multiple regression analysis using the subset of activated suppressor/cytotoxic T lymphocyte as dependent variables and the subset of activated suppression inducer/helper, helper, suppression inducer, cytotoxic and suppressor T lymphocytes, CD3, CD4 and CD8 antigen positive lymphocytes, monocytes and natural killer cells as independent variables is carried out;

c) The respective contributions of the subset of activated suppression inducer/helper,helper, suppression inducer, cytotoxic and suppressor T lymphocytes, CD3, CD4 and CD8 antigen positive lymphocytes, natural killer cells and monocytes against the subset of activated suppressor/cytotoxic T lymphocytes is determined;

d) The contributions of activated suppression inducer and activated helper T lymphocytes are calculated by allotting the contribution of the subset of activated suppression inducer/helper T lymphocytes in proportion to the ratio between the contributions of suppression inducer and helper T lymphocytes;

e) The contributions of activated suppressor and cytotoxic T lymphocytes and calculated by allotting the contribution of the subset of activated suppressor/cytotoxic T lymphocytes obtained in step b) in proportion to the ratio between the contributions of suppressor and cytotoxic T lymphocytes; and

f) The respective contributions obtained in steps d) to e) are converted into circular areas, and the respective circular areas are arranged in accordance with the mutual relationships among activated helper, activated suppression inducer, activated suppressor and activated cytotoxic T lymphocytes to draw T cell immunokinematograms.

9. A method of measuring and assessing immunokinetics through blood analysis, characterized by carrying out the following process steps (a) - (g) and assessing integrated T cell immunokinetics on the basis of the resultant B cell immunokinematograms:

a) The counts of helper, suppression inducer, cytotoxic and suppressor T lymphocytes, the subset of activated suppression inducer/helper and the subset of activated suppressor/cytotoxic T lymphocytes, OKIa1 antigen positive lymphocytes, natural killer cells, monocytes, basophils, eosinophils and neutrophils in blood samples are measured;

b) Activated B lymphocytes count is calculated by subtracting from the OKIa1 antigen positive lymphocytes count the subset of activated suppression inducer/helper T lymphocytes and the subset of activated suppressor/cytotoxic T lymphocytes counts;

c) The multiple regression analysis using activated B lymphocytes as dependent variables and helper, suppression inducer, cytotoxic, suppressor, the subset of activated suppression inducer/helper and the subset of activated suppressor/cytotoxic T lymphocytes, natural killer cells, monocytes, basophils, eosinophils and neutrophils as independent variables is carried out;

d) The respective contributions of helper, suppression inducer, cytotoxic, suppressor, the subset of activated suppression inducer/helper and the subset of activated suppressor/cytotoxic T lymphocytes, natural killer cells, monocytes, basophils, eosinophils and neutrophils against activated B lymphocytes are determined;

e) The contributions of activated helper and activated suppression inducer T lymphocytes are calculated by allotting the contribution of the subset of activated suppression inducer/helper T lymphocytes in proportion to the ratio between the contributions of helper and suppression inducer T lymphocytes;

f) The contribution of the subset of activated suppressor/cytotoxic T lymphocytes obtained in step c) is allotted in proportion to the ratio between the contributions of suppressor and cytotoxic T lymphocytes to calculate the contributions of activated suppressor and activated cytotoxic T lymphocytes; and

g) The respective contributions calculated in steps d), e) and f) except for the contributions of cytotoxic and activated cytotoxic T lymphocytes are converted into circular areas, and the respective circular areas are arranged in accordance with their immunologic mutual relationships to draw the B cell immunokinematograms.

## Patentansprüche

1. Verfahren zur Messung der Immunkinetik durch die Analyse von Blut, gekennzeichnet durch die folgenden Verfahrensschritte (a) bis (f), wodurch der Antigenerkennungsmechanismus ermittelt wird:

(a) man mißt die Zahl an Monocyten, Helfer-T-Lymphocyten und der Untergruppe an aktivierten Suppression induzierenden/Helfer-T-Lymphocyten in Blutproben;

(b) man führt die multiple Regressionsanalyse unter Verwendung von Monocyten als abhängige Variable und Helfer-T-Lymphocyten und der Untergruppe der akti vierten Suppression induzierenden/Helfer-T-Lymphocyten als unabhängige Variable durch;

(c) man bestimmt die jeweiligen partiellen Regressionskoeffizienten der Helfer-T-Lymphocyten und der Untergruppe der aktivierten Suppression induzierenden/Helfer-T-Lymphocyten und testet deren Signifikanz;

(d) man bestimmt den multiplen Korrelationskoeffizienten und testet dessen Signifikanz;

(e) man bestimmt die Beiträge der Helfer-T-Lymphocyten und der Untergruppe der aktivierten Suppression in- duzierenden/Helfer-T-Lymphocyten; und

(f) man bestimmt die Korrelation zwischen Monocyten, Helfer-T-Lymphocyten und der Untergruppe der aktivierten Suppression induzierenden/Helfer-T-Lymphocyten.

2. Verfahren zur Messung der Immunkinetik durch die Analyse von Blut, gekennzeichnet durch die folgenden Verfahrensschritte (a) bis (f), wodurch die Suppressorfunktion natürlicher Killerzellen auf Suppression induzierende T-Zellen ermittelt wird:

(a) man mißt die Zahl an natürlichen Killerzellen, Suppression induzierenden T-Lymphocyten und der Untergruppe an aktivierten Suppression induzierenden/Helfer-T-Lymphocyten in Blutproben;

(b) man führt die multiple Regressionsanalyse unter Verwendung von natürlichen Killerzellen als abhängige Variable und Suppression induzierenden T-Lymphocyten und der Untergruppe an aktivierten Suppression induzierenden/Helfer-T-Lymphocyten als unabhängige Variable durch;

(c) man bestimmt die jeweiligen partiellen Regressionskoeffizienten der Suppression induzierenden T-Lymphocyten und der Untergruppe der aktivierten Suppression induzierenden/Helfer-T-Lymphocyten und testet deren Signifikanz;

(d) man bestimmt den multiplen Korrelationskoeffizienten und testet dessen Signifikanz;

(e) man bestimmt die Beiträge der Suppression induzierenden T-Lymphocyten und der Untergruppe der aktivierten Suppression induzierenden/Helfer-T-Lymphocyten; und

(f) man bestimmt die Korrelationen zwischen natürlichen Killerzellen, Suppression induzierenden T-Lymphocyten und der Untergruppe an aktivierten Suppression induzierenden/Helfer-T-Lymphocyten.

3. Verfahren zur Messung der Immunkinetik durch die Analyse von Blut, gekennzeichnet durch die folgenden Verfahrensschritte (a) bis (f), wodurch die Funktion von natürlichen Killerzellen und Monocyten gegenüber OKla1-Antigen-positiven Lymphocyten ermittelt wird:

(a) man mißt die Zahlen an OKla1-Antigen-positiven Lymphocyten, natürlichen Killerzellen und Monocyten in Blutproben;

(b) man führt die multiple Regressionsanalyse unter Verwendung von OKla1-Antigen-positiven Lymphocyten als abhängige Variable und von natürlichen Killerzellen und Monocyten als unabhängige Variable durch;

(c) man bestimmt die jeweiligen partiellen Regressionskoeffizienten der natürlichen Killerzellen und der Monocyten und testet deren Signifikanz;

(d) man bestimmt den multiplen Korrelationskoeffizienten und testet dessen Signifikanz;

(e) man bestimmt die Beiträge der natürlichen Killerzellen und der Monocyten; und

(f) man bestimmt die Korrelationen zwischen OKla1-Antigen-positiven Lymphocyten, natürlichen Killerzellen und Monocyten.

4. Verfahren zur Messung der Immunkinetik durch die Analyse von Blut, gekennzeichnet durch die folgenden Verfahrensschritte (a) bis (f), wodurch die Funktion von natürlichen Killerzellen und Monocyten in bezug auf aktivierte Suppression induzierende/Helfer- und aktivierte Suppressor-/cytotoxische T-Lymphocyten ermittelt wird:

(a) man mißt die Zahl an aktivierten Suppression induzierenden/Helfer- und aktivierten Suppressor-/cytotoxischen T-Lymphocyten, natürlichen Killerzellen und Monocyten in Blutproben;

(b) man führt die multiple Regressionsanalyse unter Verwendung der Summe an aktivierten Suppression induzierenden/Helfer- und aktivierten Suppressor/cytotoxischen T-Lymphocyten als abhängige Variable und natürlichen Killerzellen und Monocyten als unabhängige Variable durch;

(c) man bestimmt die jeweiligen partiellen Regressionskoeffizienten der natürlichen Killerzellen und der Monocyten und testet deren Signifikanz;

(d) man bestimmt den multiplen Korrelationskoeffizienten und testet dessen Signifikanz;

(e) man bestimmt die Beiträge der natürlichen Killerzellen und der Monocyten; und

(f) man bestimmt die Korrelationen zwischen der Summe von aktivierten Suppression induzierenden/Helfer- und aktivierten Suppressor-/cytotoxischen T-Lymphocyten und natürlichen Killerzellen sowie Monocyten.

5. Verfahren zur Messung der Immunkinetik durch die Analyse von Blut, gekennzeichnet durch die folgenden Verfahrensschritte (a) bis (g), wodurch die Funktion von natürlichen Killerzellen und Monocyten in bezug auf aktivierte B-Lymphocyten ermittelt wird:

(a) man mißt die Zahl an Monocyten, natürlichen Killerzellen, OKla1-Antigen-positiven Lymphocyten und der Untergruppe der aktivierten Suppression induzierenden/Helfer-T-Lymphocyten sowie der Untergruppe der aktivierten Suppressor-/cytotoxischen T-Lymphocyten in Blutproben;

(b) man berechnet die Anzahl der aktivierten B-Lymphocyten durch Subtraktion der Zahl der Untergruppe der aktivierten Suppression induzierenden/Helfer-T-Lymphocyten und der Untergruppe der aktivierten Suppressor-/cytotoxischen T-Lymphocyten von der Zahl der OKla1-Antigen-positiven Lymphocyten;

(c) man führt die multiple Regressionsanalyse unter Verwendung von aktivierten B-Lymphocyten als abhängige Variable und natürlichen Killerzellen und Monocyten als unabhängige Variable durch;

(d) man bestimmt die jeweiligen partiellen Regressionskoeffizienten der natürlichen Killerzellen und der Monocyten und testet deren Signifikanz;

(e) man bestimmt den multiplen Korrelationskoeffizienten und testet dessen Signifikanz;

(f) man bestimmt die Beiträge der natürlichen Killerzellen und der Monocyten; und

(g) man bestimmt die Korrelationen zwischen aktivierten B-Lymphocyten und natürlichen Killerzellen sowie Monocyten.

6. Verfahren zur Messung der Immunkinetik durch die Analyse von Blut, gekennzeichnet durch die folgenden Verfahrensschritte (a) bis (d), wodurch man standardisierte partielle Regressionskoeffizienten und multiple Korrelationskoeffizienten erhält, und durch das Übertragen der Koeffizienten auf ein Koordinatensystem zur Auswertung anhand von Graphen:

(a) man mißt die Zahl an Monocyten, natürlichen Killerzellen, OKla1-Antigen-positiven Lymphocyten, Helferund Suppression induzierenden T-Lymphocyten und der Untergruppe an aktivierten Suppression induzierenden/Helfer-T-Lymphocyten in Blutproben;

(b) man bestimmt die standardisierten partiellen Regressionskoeffizienten von Helfer-T-Lymphocyten und der Untergruppe der aktivierten Suppression induzierenden/Helfer-T-Lymphocyten durch die multiple Regressionsanalyse unter Verwendung von Monocyten als abhängige Variable und Helfer-T-Lymphocyten und der Untergruppe der aktivierten Suppression induzierenden/Helfer-T-Lymphocyten als unabhängige Variable, wodurch man die Korrelationen zwischen Monocyten und Helfer-T-Lymphocyten sowie der Untergruppe der aktivierten Suppression induzierenden/Helfer-T-Lymphocyten erhält;

(c) man bestimmt die standardisierten partiellen Regressionskoeffizienten von Suppression induzierenden T-Lymphocyten und der Untergruppe der aktivierten Suppression induzierenden/Helfer-T-Lymphocyten durch die multiple Regressionsanalyse unter Verwendung von natürlichen Killerzellen als abhängige Variable und Suppression induzierenden T-Lymphocyten und der Untergruppe der aktivierten Suppression induzierenden/Helfer-T-Lymphocyten als unabhängige Variable, wodurch man die Korrelationen von natürlichen Killerzellen mit Suppression induzierenden T-Lymphocyten und der Untergruppe der aktivierten Suppression induzierenden/Helfer-T-Lymphocyten erhält; und

(d) man bestimmt die standardisierten partiellen Regressionskoeffizienten von natürlichen Killerzellen und Monocyten durch die multiple Regressionsanalyse unter Verwendung von OKIa1-Antigen-positiven Lymphocyten als abhängige Variable und natürlichen Killerzellen und Monocyten als unabhängige Variable, wodurch man die Korrelationen zwischen OKIa1-Antigen-positiven Lymphocyten und natürlichen Killerzellen sowie Monocyten erhält.

7. Verfahren zur Messung der Immunkinetik durch die Analyse von Blut, gekennzeichnet durch die folgenden Verfahrensschritte (a) bis (f), wodurch man standardisierte partielle Regressionskoeffizienten und multiple Korrelationskoeffizienten erhält, und durch das Übertragen der Koeffizienten auf ein Koordinatensystem zur Auswertung, anhand von Graphen:

(a) man mißt die Zahl an Monocyten, natürlichen Killerzellen, OKIa1-Antigen-positiven Lymphocyten, der Untergruppe der aktivierten Suppression induzierenden/Helfer-T-Lymphocyten und der Untergruppe der aktivierten Suppressor-/cytotoxischen T-Lymphocyten, sowie der Helfer- und der Suppression induzierenden T-Lymphocyten in Blutproben;

(b) man bestimmt die standardisierten partiellen Regressionskoeffizienten von Helfer-T-Lymphocyten und der Untergruppe der aktivierten Suppression induzierenden/Helfer-T-Lymphocyten durch multiple Regressionsanalyse unter Verwendung von Monocyten als abhängige Variable und von Helfer-T-Lymphocyten und der Untergruppe der aktivierten Suppression induzierenden/Helfer-T-Lymphocyten als unabhängige Variable, wodurch man die Korrelationen zwischen Monocyten und Helfer-T-Lymphocyten sowie der Untergruppe von aktivierten Suppression induzierenden/Helfer-T-Lymphocyten erhält;

(c) man bestimmt die standardisierten partiellen Regressionskoeffizienten von Suppression induzierenden T-Lymphocyten und der Untergruppe der aktivierten Suppression induzierenden/Helfer-T-Lymphocyten durch die multiple Regressionsanalyse unter Verwendung von natürlichen Killerzellen als abhängige Variable und von Suppression induzierenden T-Lymphocyten und der Untergruppe der aktivierten Suppression induzierenden/Helfer-T-Lymphocyten als unabhängige Variable, wodurch man die Korrelationen zwischen natürlichen Killerzellen und Suppression induzierenden T-Lymphocyten sowie der Untergruppe der aktivierten Suppression induzierenden/Helfer-T-Lymphocyten erhält;

(d) man bestimmt die standardisierten partiellen Regressionskoeffizienten von natürlichen Killerzellen und Monocyten durch die multiple Regressionsanalyse unter Verwendung von OKIa1-Antigen-positiven Lymphocyten als abhängige Variable und natürlichen Killerzellen und Monocyten als unabhängige Variable, wodurch man die Korrelationen zwischen OKIa1-Antigen-positiven Lymphocyten mit natürlichen Killerzellen und Monocyten erhält;

(e) man bestimmt die standardisierten partiellen Regressionskoeffizienten von natürlichen Killerzellen und Monocyten durch die multiple Regressionsanalyse unter Verwendung der Summe aus der Untergruppe der aktivierten Suppression induzierenden/Helfer-T-Lymphocyten und der Untergruppe der aktivierten Suppressor-/cytotoxischen T-Lymphocyten als abhängige Variable und von natürlichen Killerzellen und Monocyten als unabhängige Variable, wodurch man die Korrelationen zwischen aktivierten T-Lymphocyten und natürlichen Killerzellen sowie Monocyten erhält; und

(f) man berechnet die Anzahl aktivierter B-Lymphocyten durch Subtraktion der Zahl der Untergruppe der aktivierten Suppression induzierenden/Helfer-T-Lymphocyten und der Untergruppe der aktivierten Suppressor/cytotoxischen T-Lymphocyten von der Zahl der OKIa1-Antigen-positiven Lymphocyten und bestimmt die standardisierten partiellen Regressionskoeffizienten von natürlichen Killerzellen und Monocyten durch die multiple Regressionsanalyse unter Verwendung von aktivierten B-Lymphocyten als abhängige Variable und von natürlichen Killerzellen und Monocyten als unabhängige Variable, wodurch man die Korrelationen zwischen aktivierten B-Lymphocyten und natürlichen Killerzellen sowie Monocyten erhält.

8.  Verfahren zur Messung und Bewertung der Immunkinetik durch die Analyse von Blut, gekennzeichnet durch die folgenden Verfahrensschritte (a) bis (f) und die Bewertung integrierter T-Zell-Immunkinetiken auf der Basis der so erhaltenen T-Zell-Immunkinematogramme:

    (a) man mißt die Zahl an CD3-, CD4- und CD8-Antigen-positiven Lymphocyten, Helfer-, Suppression induzierenden, Suppressor- und cytotoxischen T-Lymphocyten, der Untergruppe der aktivierten Suppression induzierenden/Helfer-T-Lymphocyten und der Untergruppe der aktivierten Suppressor-/cytotoxischen T-Lymphocyten, natürlichen Killerzellen und Monocyten in Blutproben;

    (b) man führt die multiple Regressionsanalyse unter Verwendung der Untergruppe der aktivierten Suppressor/cytotoxischen T-Lymphocyten als abhängige Variable und der Untergruppe der aktivierten Suppression induzierenden/Helfer-, Helfer-, Suppression induzierenden cytotoxischen und Suppressor-T-Lymphocyten, CD3-, CD4- und CD8-Antigen-positiven Lymphocyten, Monocyten und natürlichen Killerzellen als unabhängige Variable durch;

    (c) man bestimmt die jeweiligen Beiträge der Untergruppe der aktivierten Suppression induzierenden/Helfer-, Helfer-, Suppression induzierenden, cytotoxischen und Suppressor-T-Lymphocyten, CD3-, CD4- und CD8-Antigen-positiven Lymphocyten, natürlichen Killerzellen und Monocyten gegenüber den Beiträgen der Untergruppe der aktivierten Suppressor-/cytotoxischen T-Lymphocyten;

    (d) man berechnet die Beiträge von aktivierten Suppression induzierenden und aktivierten Helfer-T-Lymphocyten durch Bestimmung des Beitrags der Untergruppe der aktivierten Suppression induzierenden/Helfer-T-Lymphocyten proportional zum Verhältnis der Beiträge von Suppression induzierenden und Helfer-T-Lymphocyten;

    (e) man berechnet die Beiträge von aktivierten Suppressor- und cytotoxischen T-Lymphocyten durch Bestimmung des Beitrags der Untergruppe der aktivierten Suppressor-/cytotoxischen T-Lymphocyten, erhalten in Schritt (b), proportional zum Verhältnis der Beiträge von Suppressor- und cytotoxischen T-Lymphocyten; und

    (f) man wandelt die in den Schritten (d) und (e) erhaltenen jeweiligen Beiträge in kreisförmige Flächen um und ordnet die jeweiligen kreisförmigen Flächen in Übereinstimmung mit den wechselseitigen Beziehungen zwischen aktivierten Helfer-, aktivierten Suppression induzierenden, aktivierten Suppressor und aktivierten cytotoxischen T-Lymphocyten zur Herstellung von T-Zell-Immunkinematogrammen an.

9.  Verfahren zur Messung und Bewertung der Immunkinetik durch die Analyse von Blut, gekennzeichnet durch die folgenden Verfahrensschritte (a) bis (g) und die Bewertung integrierter T-Zell-Immunkinetiken auf der Basis der so erhaltenen B-Zell-Immunkinematogramme:

    (a) man mißt die Zahl an Helfer-, Suppression induzierenden, cytotoxischen und Suppressor-T-Lymphocyten, der Untergruppe der aktivierten Suppression induzierenden/Helfer-T-Lymphocyten und der Untergruppe der aktivierten Suppressor-/cytotoxischen T-Lymphocyten, OKIa1-Antigen-positiven Lymphocyten, natürlichen Killerzellen, Monocyten, Basophilen, Eosinophilen und Neutrophilen in Blutproben;

    (b) man berechnet die Anzahl an aktivierten B-Lymphocyten durch Subtraktion der Zahl der Untergruppe der aktivierten Suppression induzierenden/Helfer-T-Lymphocyten und der Untergruppe der aktivierten Suppressor-/cytotoxischen T-Lymphocyten von der Zahl der OKIa1-Antigen-positiven Lymphocyten;

    (c) man führt die multiple Regressionsanalyse unter Verwendung von aktivierten B-Lymphocyten als abhängige Variable und von Helfer-, Suppression induzierenden, cytotoxischen, Suppressor-T-Lymphocyten, der Untergruppe der aktivierten Suppression induzierenden/Helfer- und der Untergruppe der aktivierten Suppressor-/cytotoxischen T-Lymphocyten, natürlichen Killerzellen, Monocyten, Basophilen, Eosinophilen und Neutrophilen als unabhängige Variable durch;

    (d) man bestimmt die jeweiligen Beiträge von Helfer-, Suppression induzierenden, cytotoxischen, Suppressor-T-Lymphocyten, der Untergruppe der aktivierten Suppression induzierenden/Helfer- und der Untergruppe der aktivierten Suppressor-/cytotoxischen T-Lymphocyten, natürlichen Killerzellen, Monocyten, Basophilen, Eosinophilen und Neutrophilen gegenüber aktivierten B-Lymphocyten;

    (e) man berechnet die Beiträge von aktivierten Helferund aktivierten Suppression induzierenden T-Lymphocyten durch Bestimmung des Beitrags der Untergruppe der aktivierten Suppression induzierenden/Helfer-T-Lymphocyten proportional zum Verhältnis der Beiträge von Helfer- und Suppression induzierenden T-Lymphocyten;

    (f) man bestimmt den Beitrag der Untergruppe der aktivierten Suppressor-/cytotoxischen T-Lymphocyten, erhalten in Schritt (c), proportional zum Verhältnis der Beiträge von Suppressor- und cytotoxischen T-Lymphocyten, um die Beiträge von aktivierten Suppressor- und aktivierten cytotoxischen T-Lymphocyten zu berechnen; und

(g) man wandelt die jeweiligen, in den Schritten (d), (e) und (f) errechneten Beiträge mit Ausnahme der Beiträge der cytotoxischen und der aktivierten cytotoxischen T-Lymphocyten in kreisförmige Flächen um und ordnet die jeweiligen kreisförmigen Flächen in Übereinstimmung mit ihren immunologischen wechselseitigen Beziehungen zur Erstellung von B-Zell-Kinematogrammen an.

## Revendications

1. Procédé de mesures immunocinétiques par analyse sanguine caractérisé par la réalisation des étapes de procédé (a) à (f) suivantes pour établir le mécanisme de reconnaissance antigénique :

(a) les numérations des monocytes, lymphocytes T helper et du sous-ensemble des lymphocytes T inducteurs de suppression/helper activés dans les échantillons sanguins sont mesurées;

(b) l'analyse de régression multiple avec les monocytes comme variables dépendantes et les lymphocytes T helper et le sous-ensemble des inducteurs de suppression/helper activés comme variables indépendantes est réalisée;

(c) les coefficients respectifs partiels de régression des lymphocytes T helper et du sous-ensemble des inducteurs de suppression/helper activés sont déterminés et les significations sont testées;

(d) le coefficient de corrélation multiple est déterminé et la signification est testée;

(e) les contributions des lymphocytes T helper et du sous-ensemble des inducteurs de suppression/helper activés sont déterminées, et

(f) les corrélations entre les monocytes, les lymphocytes T helper et le sous-ensemble des lymphocytes T inducteur de suppression/helper activés sont déterminées.

2. Procédé de mesures immunocinétiques par analyse sanguine, caractérisé par la réalisation des étapes de procédé (a) à (f) suivantes pour établir la fonction suppressive des cellules tueuses naturelles sur les lymphocytes T inducteurs de suppression :

(a) les numérations des cellules tueuses naturelles, des lymphocytes T inducteurs de suppression et du sous-ensemble des lymphocytes T inducteurs de suppression/helper activés dans les échantillons sanguins sont mesurées;

(b) l'analyse de régression multiple avec les cellules tueuses naturelles comme variables dépendantes et les lymphocytes T inducteurs de suppression et le sous-ensemble des inducteurs de suppression/helper activés comme variables indépendantes est réalisée;

(c) les coefficients respectifs partiels de régression des lymphocytes T inducteurs de suppression et du sous-ensemble des inducteurs de suppression/helper activés sont déterminés et les significations sont testées;

(d) le coefficient de corrélation multiple est déterminé et la signification est testée;

(e) les contributions des lymphocytes T inducteurs de suppression et du sous-ensemble des inducteurs de suppression/helper activés sont déterminées, et

(f) les corrélations entre les cellules tueuses naturelles, les lymphocytes T inducteurs de suppression et le sous-ensemble des lymphocytes T inducteurs de suppression/helper activés sont déterminées.

3. Procédé de mesures immunocinétiques par analyse sanguine, caractérisé par la réalisation des étapes de procédé (a) à (f) suivantes pour établir la fonction des cellules tueuses naturelles et des monocytes vis-à-vis des lymphocytes positifs à l'antigène OKIa1 :

(a) les numérations des lymphocytes positifs à l'antigène OKIa1, des cellules tueuses naturelles et des monocytes dans les échantillons sanguins sont mesurées;

(b) l'analyse de régression multiple avec les lymphocytes positifs à l'antigène OKIa1 comme variables dépendantes et les cellules tueuses naturelles et les monocytes comme variables indépendantes est réalisée;

(c) les coefficients respectifs partiels de régression des cellules tueuses naturelles et des monocytes sont déterminés et les significations sont testées;

(d) le coefficient de corrélation multiple est déterminé et la signification est testée;

(e) les contributions des cellules tueuses naturelles et des monocytes sont déterminées, et

(f) les corrélations entre les lymphocytes positifs à l'antigène OKIa1, les cellules tueuses naturelles et les monocytes sont déterminées.

4. Procédé de mesures immunocinétiques par analyse sanguine, caractérisé par la réalisation des étapes de procédé (a) à (f) suivantes pour établir la fonction des cellules tueuses naturelles et des monocytes sur les lymphocytes T inducteurs de suppression/helper activés et les suppresseurs/cytotoxiques activés :

(a) les numérations des lymphocytes T inducteurs de suppression/helper activés, des lymphocytes T suppresseurs/cytotoxiques activés, des cellules tueuses naturelles et des monocytes dans les échantillons sanguins sont mesurées;

(b) l'analyse de régression multiple avec la somme des lymphocytes T inducteurs de suppression/helper activés et des suppresseurs/cytotoxiques activés comme variables dépendantes et les cellules tueuses naturelles et les monocytes comme variables indépendantes est réalisée;

(c) les coefficients respectifs partiels de régression des cellules tueuses naturelles et des monocytes sont déterminés et les significations sont testées;

(d) le coefficient de corrélation multiple est déterminé et la signification est testée;

(e) les contributions des cellules tueuses naturelles et des monocytes sont déterminées, et

(f) les corrélations de la somme des lymphocytes T inducteurs de suppression/helper activés et des suppresseurs/cytotoxiques activés avec les cellules tueuses naturelles et les monocytes sont déterminées.

5. Procédé de mesures immunocinétiques par analyse sanguine, caractérisé par la réalisation des étapes de procédé (a) à (g) suivantes pour établir la fonction des cellules tueuses naturelles et des monocytes sur les lymphocytes B activés :

(a) les numérations des monocytes, des cellules tueuses naturelles, des lymphocytes positifs à l'antigène OKIa1, du sous-ensemble des lymphocytes T inducteurs de suppression/helper activés et du sous-ensemble des lymphocytes T suppresseurs/cytotoxiques activés dans les échantillons sanguins sont mesurées;

(b) on calcule la numération des lymphocytes B activés en soustrayant de la numération des lymphocytes positifs à l'antigène OKIa1, celle du sous-ensemble des lymphocytes T inducteurs de suppression/helper activés et celle du sous-ensemble des lymphocytes T suppresseurs/cytotoxiques activés;

(c) l'analyse de régression multiple avec les lymphocytes B activés comme variables dépendantes et les cellules tueuses naturelles et les monocytes comme variables indépendantes est réalisée;

(d) les coefficients respectifs partiels de régression des cellules tueuses naturelles et des monocytes sont déterminés et les significations sont testées;

(e) le coefficient de corrélation multiple est déterminé et la signification est testée;

(f) les contributions des cellules tueuses naturelles et des monocytes sont déterminées, et

(g) les corrélations des lymphocytes B activés avec les cellules tueuses naturelles et les monocytes sont déterminées.

6. Procédé de mesures immunocinétiques par analyse sanguine, caractérisé par la réalisation des étapes de procédé (a) à (d) suivantes pour obtenir les coefficients partiels standardisés de régression et les coefficients de corrélation multiples et inscrire les coefficients comme coordonnées pour préparer des graphiques pour évaluation :

(a) les numérations des monocytes, des cellules tueuses naturelles, des lymphocytes positifs à l'antigène OKIa1, des lymphocytes T helper et inducteurs de suppression et du sous-ensemble des lymphocytes T inducteurs de suppression/helper activés dans les échantillons sanguins sont mesurées;

(b) les coefficients partiels standardisés de régression des lymphocytes T helper et du sous-ensemble des inducteurs de suppression/helper activés sont déterminés par l'analyse de régression multiple avec les monocytes comme variables dépendantes et les lymphocytes T helper et le sous-ensemble des inducteurs de suppression/helper activés comme variables indépendantes, pour obtenir les corrélations des monocytes avec les lymphocytes T helper et le sous-ensemble des inducteurs de suppression/helper activés;

(c) les coefficients partiels standardisés de régression des lymphocytes T inducteurs de suppression et du sous-ensemble des inducteurs de suppression/helper activés sont déterminés par l'analyse de régression multiple avec les cellules tueuses naturelles comme variables dépendantes et les lymphocytes T inducteurs de suppression et le sous-ensemble des inducteurs de suppression/helper activés comme variables indépendantes, pour obtenir les corrélations des cellules tueuses naturelles avec les lymphocytes T inducteurs de suppression et le sous-ensemble des inducteurs de suppression/helper activés, et

(d) les coefficients partiels standardisés de régression des cellules tueuses naturelles et des monocytes sont déterminés par l'analyse de régression multiple avec les lymphocytes positifs à l'antigène OKIa1 comme variables dépendantes et les cellules tueuses naturelles et les monocytes comme variables indépendantes, pour obtenir les corrélations des lymphocytes positifs à l'antigène OKIa1 avec les cellules tueuses naturelles et les monocytes.

7. Procédé de mesures immunocinétiques par analyse sanguine, caractérisé par la réalisation des étapes de procédé (a) à (f) suivantes pour obtenir les coefficients partiels standardisés de régression et les coefficients de corrélation multiples et inscrire les coefficients comme coordonnées pour préparer des graphiques pour évaluation :

(a) les numérations des monocytes, des cellules tueuses naturelles, des lymphocytes positifs à l'antigène ORIa1, du sous-ensemble des lymphocytes T inducteurs de suppression/helper activés, du sous-ensemble des lymphocytes T suppresseurs/cytotoxiques activés et des lymphocytes T helper et inducteurs de suppression dans les échantillons sanguins sont mesurées;

(b) les coefficients partiels standardisés de régression des lymphocytes T helper et du sous-ensemble des inducteurs de suppression/helper activés sont déterminés par l'analyse de régression multiple avec les monocytes comme variables dépendantes et les lymphocytes T helper et le sous-ensemble des inducteurs de suppression/helper activés comme variables indépendantes, pour obtenir les corrélations des monocytes avec les lymphocytes T helper et le sous-ensemble des inducteurs de suppression/helper activés;

(c) les coefficients partiels standardisés de régression des lymphocytes T inducteurs de suppression et du sous-ensemble des inducteurs de suppression/helper activés sont déterminés par l'analyse de régression multiple avec les cellules tueuses naturelles comme variables dépendantes et les lymphocytes T inducteurs de suppression et le sous-ensemble des inducteurs de suppression/helper activés comme variables indépendantes, pour obtenir les corrélations des cellules tueuses naturelles avec les lymphocytes T inducteurs de suppression et le sous-ensemble des inducteurs de suppression/helper activés;

(d) les coefficients partiels standardisés de régression des cellules tueuses naturelles et des monocytes sont déterminés par l'analyse de régression multiple avec les lymphocytes positifs à l'antigène ORIa1 comme variables dépendantes et les cellules tueuses naturelles et les monocytes comme variables indépendantes, pour obtenir les corrélations des lymphocytes positifs à l'antigène OKIa1 avec les cellules tueuses naturelles et les monocytes;

(e) les coefficients partiels standardisés de régression des cellules tueuses naturelles et des monocytes sont déterminés par l'analyse de régression multiple avec la somme du sous-ensemble des lymphocytes T inducteurs de suppression/helper activés et du sous-ensemble des suppresseurs/cytotoxiques activés comme variables dépendantes et les cellules tueuses naturelles et les monocytes comme variables indépendantes, pour obtenir les corrélations des lymphocytes T activés avec les cellules tueuses naturelles et les monocytes, et

(f) on calcule la numération des lymphocytes B activés en soustrayant les numérations du sous-ensemble des lymphocytes T inducteurs de suppression/helper activés et du sous-ensemble des suppresseurs/cytotoxiques activés de la numérotation des lymphocytes positifs à l'antigène OKIa1, et les coefficients partiels standardisés de régression des cellules tueuses naturelles et des monocytes sont déterminés par l'analyse de régression multiple avec les lymphocytes B activés comme variables dépendantes et les cellules tueuses naturelles et les monocytes comme variables indépendantes, pour obtenir les corrélations des lymphocytes B activés avec les cellules tueuses naturelles et les monocytes.

8. Procédé de mesures et d'évaluations immunocinétiques par analyse sanguine, caractérisé par la réalisation des étapes de procédé (a) à (f) suivantes et l'évaluation des immunocinétiques intégrées des cellules T sur base des immunocinématogrammes résultants des cellules T :

(a) les numérations des lymphocytes positifs aux antigènes CD3, CD4 et CD8, des lymphocytes T helper, inducteurs de suppression, suppresseurs et cytotoxiques, du sous-ensemble des lymphocytes T inducteurs de suppression/helper activés et du sous-ensemble des lymphocytes T suppresseurs/cytotoxiques activés, des cellules tueuses naturelles et des monocytes dans les échantillons sanguins sont mesurées;

(b) l'analyse de régression multiple avec le sous-ensemble des lymphocytes T suppresseurs/cytotoxiques activés comme variables dépendantes et le sous-ensemble des lymphocytes T inducteurs de suppression/helper activés, les lymphocytes T helper, inducteurs de suppression, cytotoxiques et suppresseurs, les lymphocytes positifs aux antigènes CD3, CD4 et CD8, les monocytes et les cellules tueuses naturelles comme variables indépendantes est réalisée;

(c) les contributions respectives du sous-ensemble des lymphocytes T inducteurs de suppression/helper activés, des lymphocytes T helper, inducteurs de suppression, suppresseurs et cytotoxiques, des lymphocytes positifs aux antigènes CD3, CD4 et CD8, des cellules tueuses naturelles et des monocytes vis-à-vis du sous-ensemble des lymphocytes T suppresseurs/cytotoxiques activés sont déterminées;

(d) les contributions des lymphocytes T inducteurs de suppression activés et helper activés sont calculées en distribuant la contribution du sous-ensemble des lymphocytes T inducteurs de suppression/helper activés en proportion du rapport entre les contributions des lymphocytes T inducteurs de suppression et helper;

(e) les contributions des lymphocytes T suppresseurs activés et cytotoxiques sont calculées en distribuant la contribution du sous-ensemble des lymphocytes T suppresseurs/cytotoxiques activés obtenue à l'étape (b) en proportion du rapport entre les contributions des lymphocytes T suppresseurs et cytotoxiques, et

(f) les contributions respectives obtenues aux étapes (d) et (e) sont converties en surfaces circulaires et les surfaces circulaires respectives sont placées suivant les relations mutuelles entre les lymphocytes T helper activés, inducteurs de suppression activés, suppresseurs activés et cytotoxiques activés pour dessiner les immunocinématogrammes de cellules T

9. Procédé de mesures et d'évaluations immunocinétiques par analyse sanguine, caractérisé par la réalisation des étapes de procédé (a) à (g) suivantes et l'évaluation des immunocinétiques intégrées des cellules T sur base des immunocinématogrammes résultants des cellules B :

(a) les numérations des lymphocytes T helper, inducteurs de suppression, suppresseurs et cytotoxiques, du sous-ensemble des lymphocytes T inducteurs de suppression/helper activés et du sous-ensemble des lymphocytes T suppresseurs/cytotoxiques activés, des lymphocytes positifs à l'antigène OKIa1, des cellules tueuses naturelles et des monocytes, des basophiles, des éosinophiles et des neutrophiles dans les échantillons sanguins sont mesurées;

(b) on calcule la numération des lymphocytes B activés en soustrayant de la numération des lymphocytes positifs à l'antigène OKIa1, les numérations du sous-ensemble des lymphocytes T inducteurs de suppression/helper activés et du sous-ensemble des lymphocytes T suppresseurs/cytotoxiques activés;

(c) l'analyse de régression multiple avec les lymphocytes B activés comme variables dépendantes et les lymphocytes T helper, inducteurs de suppression, suppresseurs et cytotoxiques, le sous-ensemble des lymphocytes T inducteurs de suppression/helper activés et le sous-ensemble des lymphocytes T suppresseurs/cytotoxiques activés, les cellules tueuses naturelles, les monocytes, les basophiles, les éosinophiles et les neutrophiles comme variables indépendantes est réalisée;

(d) les contributions respectives des lymphocytes T helper, inducteurs de suppression, suppresseurs et cytotoxiques, du sous-ensemble des lymphocytes T inducteurs de suppression/helper activés et du sous-ensemble des lymphocytes T suppresseurs/cytotoxiques activés des cellules tueuses naturelles, des monocytes, des basophiles, des éosinophiles et des neutrophiles vis-à-vis des lymphocytes B activés sont déterminées;

(e) les contributions des lymphocytes T inducteurs de suppression activés et helper activés sont calculées en distribuant la contribution du sous-ensemble des lymphocytes T inducteurs de suppression/helper activés en proportion du rapport entre les contributions des lymphocytes T inducteurs de suppression et helper;

(f) la contribution du sous-ensemble des lymphocytes T suppresseurs/cytotoxiques activés obtenue à l'étape (c) est distribuée en proportion du rapport entre les contributions des lymphocytes T suppresseurs et cytotoxiques pour calculer les contributions des lymphocytes T suppresseurs activés et cytotoxiques activés, et

(g) les contributions respectives calculées aux étapes (d), (e) et (f) sauf pour les contributions des lymphocytes T cytotoxiques et cytotoxiques activés sont converties en surfaces circulaires et les surfaces circulaires respectives sont placées suivant leurs relations mutuelles immunologiques pour dessiner les immunocinématogrammes des cellules B.

FIG. 1

normal control group

Monocyte
Act.si/hT (11.83%)
F value (p<0.05)

NK cell
Act.si/hT (2.62%)
F value (n.s.)

progressive disease group

Monocyte
Act.si/hT (0.06%)
F value (p<0.05)

NK cell
Act.si/hT (25.69%)
F value (p<0.01)

stable or improved group

Monocyte
Act.si/hT (0.08%)
F value (p<0.05)

NK cell
Act.si/hT (15.96%)
F value (p<0.01)

FIG. 2

FIG. 3

◎ Monocyte vs. Th and Act.si/hT (A)
☐ NK cell vs. Tsi and Act.si/hT (B)
○ OKIal vs. Monocyte and NK cell (C)

(Monocyte, Th, Tsi)

$p < 0.05$    $**p < 0.01$

FIG. 4

◎  Monocyte vs. Th and Act.si/hT   (A)
▢  NK cell vs. Tsi and Act.si/hT   (B)
◯  OKIal vs. Monocyte and NK cell   (C)

(Monocyte, Th, Tsi)

——— normal control group (n=40)
——— recurrent group        (n=27)
········ not-recurrent group  (n=32)

0.535[**]

0.5

○0.570[**]

0.323

▢0.624[**]

0.362

0.205

▢0.19?

0.5

0

0.100

0.218

(Act.si/hT, NK cell)

⋅ p < 0.0 5   ⋅⋅ p < 0.0 1

FIG. 5

EP 0 443 036 B1

Multiple Correlation
Coefficient

◎ Monocyte vs. Th and Act.si/hT (A)
□ NK cell vs. Tsi and Act.si/hT (B)
○ OKIal vs. Monocyte and NK cell (C)

━━━ normal control group        (n=40)
──── progressive disease group  (n=50)
─·─· stable or improved group   (n=93)

(Monocyte, Th, Tsi)

1.0

0.5

0.5

-0.25

0

* p < 0.05

** p < 0.01

-0.25

0.5

(Act.si/hT, NK cell)

FIG. 6(A)

EP 0 443 036 B1

78

normal control group (n=40)

FIG. 6(B)

progressive disease group (n=50)

stable or improved group (n=93)

△ Monocyte vs. Th and Act.si/hT (A)

□ NK cell vs. Tsi and Act.si/hT (B)

● OKIal vs. Monocyte and Act.si/hT (C)

○ Activated T Lymphocyte vs. Monocyte and NK cell (D)

◉ Activated B Lymphocyte vs. Monocyte and NK cell (E)

* $p < 0.05$ (f-test)

** $p < 0.01$

(n=40)

r=0.92893 (P<0.01)

FIG. 7

$r = 0.998937$ $(P < 0.05)$
$(n = 13)$

OKT3

OKT4  OKT8  OKT4

Th  Tc  Ts  Tsl

Monocyte  Mφ  Act. Th  Act. Tc  Act. Ts  Act. Tsl

(1.33%)  HX  (11.03%)

$r = 0.861425$ $(n. s.)$
$(n = 13)$

FIG. 8

−0.5  0.5  0.5  Y

Z

0

−0.5  0.5  X

Th  Ts  Tsl

Monocyte  Mφ  Act. Th  Act. B  Act. Ts  Act. Tsl

(1.33%)  Baso  (0.05%)  HX  (11.03%)

(1.41%)  Eos  Seg

(0.75%)  (0.04%)

81

FIG. 9